# EUROPEAN PATENT APPLICATION

(11) **EP 4 681 814 A1**
(43) Date of publication of application: **21.01.2026**
(21) Application number: 25194870.9
(22) Date of filing: 24.05.2022
(51) Int. Cl.: B01J 20/289, B01D 15/38, B01J 20/32, B01J 20/34

(54) **SCAFFOLD FOR ISOLATION OF A BIOMOLECULE**

(30) Priority: 24.05.2021 GB 202107352
(62) Divisional of application: 22729752.0
(71) Applicant: Astrea UK Services Limited, Cambridge Cambridgeshire CB23 7AJ (GB)
(72) Inventor: BURTON, Steve, Cambridge, CB23 7AJ (GB); SADLER, Chris, Cambridge, CB23 7AJ (GB)
(74) Representative: Petty, Catrin Helen

(57) **Abstract**

A method of producing an activated substrate comprising:
(a) modifying a substrate, wherein the substrate comprises a base matrix, to form a base matrix comprising a leaving group;
(b) contacting the base matrix formed in step (a) with an aminating agent, to thereby provide an aminated base matrix; and
(c) contacting the aminated base matrix formed in step (b) with a heteroaromatic compound, wherein the heteroaromatic compound is a 5 to 12 membered heteroaromatic ring substituted with at least two halogens and optionally one or more further substituents, to produce an activated substrate.

The activated substrate may be contacted with a molecule comprising a ligand specific for a biomolecule to provide a scaffold for isolation of the biomolecule.

## Description

The present invention is concerned with methods of producing an activated substrate and a scaffold for isolation of a biomolecule. The invention is also concerned with the activated substrate and the scaffold *per se.* Finally, the application discloses a method of using the scaffold to isolate a biomolecule, for example in affinity chromatography.

Isolation of highly pure biomolecules is a requirement of a number of fields including biotechnology applications and the manufacture of biopharmaceutical products. Chromatography is used extensively in industry for purification of biopharmaceuticals including therapeutic proteins, nucleic acids, and cells etc. The downstream process for purification of biomolecules using traditional chromatography and alternative isolation methods such as the Cohn Process is inefficient and costly especially at large scale. The use of affinity chromatography in the purification of target biomolecules affords potential for far greater specificity than traditional separation methods allowing more efficient and cost-effective downstream bioprocesses.

WO 2018/011600 discloses a process for preparing a functionalised polymeric chromatography medium, including a functionalisation in which:
(a) a grafted product is first contacted with a reagent selected from the group selected from divinyl sulfone, allyl glycidyl ether, and combinations thereof;
(b) the product of step (a) is optionally treated with a halohydrin-forming reagent or an epoxide-forming reagent; and
(c) the product of step (b) is contacted with a Protein A.

WO00/67900 discloses triazine-based detoxification agents.

The performance of affinity adsorbents on repeated use in terms of maintained selectivity and capacity is dependent on cleaning the solid phase matrix to restore it ready for subsequent use by means of a clean in place (CIP) step in the chromatography process. Sanitisation of the matrix is also necessary to avoid contamination from bacteria, viruses, and endotoxin. The conditions typically used for this cleaning and sanitisation is aqueous NaOH (0.1 M to 1.0 M NaOH) which means exposure of the matrix to solutions in excess of pH 13. NaOH is favoured for this purpose as it is cheap, non-toxic, freely available, and relatively easy to dispose of.

However, high pH conditions used in CIP and sanitisation steps are detrimental to the stability of some affinity ligands, notably protein-based ligands. High pH can affect the tertiary structure of the affinity ligand and in some cases cleavage of the ligand by hydrolysis which may diminish the performance of the adsorbent. This degradation can limit the re-use potential of an adsorbent and therefore increase the cost of bioprocesses and in turn the products manufactured with them.

Affinity ligands which have improved caustic stability are known in the art. The modification of asparagine residues in protein ligands to improve alkali stability is discussed in US 6,831,161 B1. Patent WO 03/080655 also discusses the mutation of asparagine residues to amino acids other than glutamine or aspartic acid in immunoglobulin binding protein ligands to improve caustic stability. The use of functional protein fragments such as domain C from Staphylococcus protein A to provide adsorbents with improved stability to high pH is captured in US20140135476A1. Use of peptide ligands with improved caustic stability compared to protein-based ligands for immunoglobulin purification is described in US9162223B2.

The availability of highly stable, cost effective affinity chromatography adsorbents with low toxicological risk is therefore of particular interest in bioprocessing to achieve affordable therapeutics.

Further, it is desirable for activated adsorbents used for covalent attachment of affinity ligands to possess a high density of chemically reactive groups. High activation densities on solid supports afford increased reaction rates for ligand coupling which in turn allows reduction in ligand concentration required to drive the coupling reaction, manufacturing process time and reduction in reaction temperatures. This is particularly important for costly ligands and fragile ligands that require mild immobilisation reaction conditions to avoid degradation. Another advantage to high activation density is achievement of higher immobilised ligand concentrations. This affords the possibility of increasing the binding capacity for target biomolecules. Triazine adsorbents disclosed in the prior art, for example WO2004052870A1, describe the use of activating agents such as epichlorohydrin for epoxide activation. This type of activation chemistry, whist offering caustic stability, is limited in terms of the possible density of chemically reactive groups achievable on beaded agarose supports.

The present invention arises from the inventors' attempts to overcome the problems associated with the prior art.

Therefore, in accordance with a first aspect of the invention, there is provided a method of producing an activated substrate, the method comprising:
(a) modifying a substrate, wherein the substrate comprises a base matrix, to form a base matrix comprising a leaving group and/or an optionally substituted 3 membered heterocyclic ring;
(b) contacting the base matrix formed in step (a) with an aminating agent, to thereby provide an aminated base matrix; and
(c) contacting the aminated base matrix formed in step (b) with a heteroaromatic compound, wherein the heteroaromatic compound is a 5 to 12 membered heteroaromatic ring substituted with at least two halogens and optionally one or more further substituents, to produce an activated substrate.

The activated substrate, also referred to herein as the activated base matrix, may be understood to be activated in that it is configured to further react with a molecule comprising a ligand to provide a scaffold for isolation of a biomolecule. Advantageously, therefore, the method provides n activated substrate, which can be further reacted with a molecule comprising a ligand to provide a scaffold for isolation of a biomolecule.

Activation of a substrate as described herein may achieve a high surface concentration of chemically active groups which may be conveniently converted to a high concentration of more highly reactive groups

The substrate may be a solid support. The solid support may be selected from the group consisting of a controlled pore glass, a magnetic controlled pore glass, a silicacontaining particle, a polymer, and a controlled pore glass grafted with a polymer. The solid support may comprise a polymer. The polymer may be a natural polymer or a synthetic polymer. The polymer may be a polysaccharide, a polymethacrylate, a polymer of styrene, a copolymer of styrene and divinylbenzene, a copolymer of styrene and divinylbenzene grafted with polyethyleneglycol or a copolymer of dimethylacrylamide and N,N,-bisacryloylethylenediamine. The polysaccharide may be agarose, cellulose, hemicellulose, dextran, carrageenan or chitin.

The substrate may be a fibre, a fibre mat, a membrane, a solid bead, a porous bead, a monolith or a solid gel.

Preferably, the substrate comprises a nucleophilic moiety. The nucleophilic moiety may be an -OH, SH or -NH₂ group. Preferably, the nucleophilic moiety is a hydroxyl moiety.

The method preferably comprises modifying the substrate to form a base matrix comprising a leaving group. The leaving group may be a halogen.

Modifying the substrate to form a base matrix comprising a leaving group may comprise:
ai) contacting the substrate with an electrophile, wherein the electrophile comprises an unsaturated hydrocarbon chain, to thereby form a base matrix comprising an unsaturated hydrocarbon chain; and
aii) contacting the base matrix formed in step (ai) with a halogenating agent, to thereby provide a halogenated base matrix.

Alternatively, the method may comprise modifying the substrate to form a base matrix comprising an optionally substituted heterocyclic ring. may be a 3 membered ring. The optionally substituted heterocyclic ring may be an optionally substituted epoxide.

Modifying the substrate to form a base matrix comprising an optionally substituted heterocyclic ring comprises contacting the substrate with an electrophile, wherein the electrophile comprises an optionally substituted heterocyclic ring, to thereby provide a base matrix comprising an optionally substituted heterocyclic ring.

Accordingly, both of the above methods comprise contacting the substrate with an electrophile.

The electrophile may comprise 1 to 24 carbon atoms, more preferably the electrophile comprises 2 to 12 carbon atoms or 3 to 10 carbon atoms, and most preferably 4 to 8 carbon atoms, or 5 to 7 carbon atoms.

The electrophile may comprise a C₂₋₁₂ unsaturated hydrocarbon chain, more preferably a C₂₋₆ unsaturated hydrocarbon chain, and most preferably a C₂₋₃ unsaturated hydrocarbon chain. The unsaturated hydrocarbon chain may be an allyl group.

The electrophile may comprise an ether group.

The electrophile may be a compound of formula (I):

R¹-L¹-X¹-L²-R² (I)

, wherein R¹ is an optionally substituted 3 membered heterocyclic ring or a leaving group;
R² is an optionally substituted C₂-C₁₂ alkenyl, an optionally substituted C₂-C₁₂ alkynyl or an optionally substituted 3 membered heterocyclic ring, preferably a C₂-C₁₂ alkenyl, an optionally substituted C₂-C₁₂ alkynyl;
L¹ and L² are each independently absent, an optionally substituted C₁₋₁₂ alkylene, an optionally substituted C₂₋₁₂ alkenylene or an optionally substituted C₂₋₁₂ alkynylene, where the backbone of the alkylene, alkenylene or alkynlene is optionally interrupted by one or more heteroatoms;
X¹ is NR³, O or S, preferably O; and
R³ is H, an optionally substituted C₁₋C₆ alkyl, an optionally substituted C₂-C₆ alkenyl or an optionally substituted C₂-C₆ alkynyl.

The term "alkyl", as used herein, unless otherwise specified, refers to a saturated straight or branched hydrocarbon. An alkyl group can be unsubstituted or substituted with one or more of halogen, OH, SH, COOH, NH₂ or oxo.

"Alkenyl" refers to olefinically unsaturated hydrocarbon groups which can be unbranched or branched. An alkenyl group can be unsubstituted or substituted with one or more of halogen, OH, SH, COOH, NH₂, oxo or an optionally substituted C₁₋₆ alkynyl.

"Alkynyl" refers to acetylenically unsaturated hydrocarbon groups which can be unbranched or branched. An alkenyl group can be unsubstituted or substituted with one or more of halogen, OH, SH, COOH, NH₂, oxo or an optionally substituted C₁₋₆ alkenyl.

The term "alkylene", as used herein, unless otherwise specified, refers to a bivalent saturated straight or branched hydrocarbon. An alkylene group can be unsubstituted or substituted with one or more of halogen, OH, SH, NH₂, COOH or oxo.

The term "alkenylene", as used herein, unless otherwise specified, refers to a bivalent olefinically unsaturated straight or branched hydrocarbon. An alkenylene group can be unsubstituted or substituted with one or more of halogen, OH, SH, COOH, NH₂, oxo or an optionally substituted C₁₋₆ alkynyl.

The term "alkynylene", as used herein, unless otherwise specified, refers to a bivalent acetylenically unsaturated straight or branched hydrocarbon. An alkynylene group can be unsubstituted or substituted with one or more of halogen, OH, SH, COOH, NH₂, oxo or an optionally substituted C₁₋₆ alkenyl.

A "heterocyclic ring" may refer to 3 membered monocyclic rings in which at least one ring atom is a heteroatom. The or each heteroatom may be independently selected from the group consisting of oxygen, sulfur and nitrogen. A heterocyclic ring may be saturated or partially saturated. A heterocyclic group can be unsubstituted or substituted with one or more of halogen, OH, SH, COOH, NH₂, oxo, optionally substituted C₁-C₆ alkyl, optionally substituted C₂-C₆ alkenyl or optionally substituted C₂-C₆ alkynyl.

"Heteroaryl" and "heteroaromatic ring" refers to a monocyclic or fused, e.g. bicyclic, aromatic 5 to 10 membered ring system in which at least one ring atom is a heteroatom. The or each heteroatom may be independently selected from the group consisting of oxygen, sulfur and nitrogen.

"Aryl", "arylene" and "aromatic ring" refers to a monocyclic or fused, e.g. bicyclic, aromatic 6-12 membered aromatic ring system.

An optionally substituted aromatic or heteroaromatic group as described anywhere herein can be unsubstituted or substituted with one or more of halogen, OH, SH, COOH, NH₂, oxo, optionally substituted C₁-C₆ alkyl, optionally substituted C₂-C₆ alkenyl or optionally substituted C₂-C₆ alkynyl.

A "heteroatom" may be O, NR⁴ or S, wherein R⁴ is H, an optionally substituted C₁₋C₆ alkyl, an optionally substituted C₂-C₆ alkenyl or an optionally substituted C₂-C₆ alkynyl.

A "halogen" may be fluorine, chlorine, bromine or iodine.

R¹ may be an optionally substituted 3 membered heterocyclic ring.

Accordingly, R¹ may be 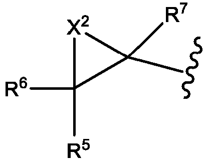
, wherein R⁵, R⁶ and R⁷ are independently H, OR⁸, COOR⁸, NR⁸R⁹, a halogen, an optionally substituted C₁₋C₆ alkyl, an optionally substituted C₂-C₆ alkenyl or an optionally substituted C₂-C₆ alkynyl;
X² is NR¹⁰, O or S; and
R⁸ to R¹⁰ are independently H, an optionally substituted C₁₋C₆ alkyl, an optionally substituted C₂-C₆ alkenyl or an optionally substituted C₂-C₆ alkynyl.

Accordingly, in a preferred embodiment, the compound of formula (I) is a compound of formula (Ia):

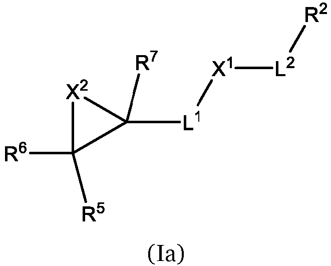

Preferably, R⁵, R⁶ and R⁷ are independently H, a C₁-C₃ alkyl, a C₂-C₃ alkenyl or a C₂-C₃ alkynyl. Preferably, R⁵, R⁶ and R⁷ are each H.

X² is preferably O.

Accordingly, in a more preferred embodiment, the compound of formula (I) is a compound of formula (Iai):

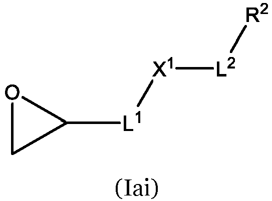

In embodiments where R¹ is a leaving group, the leaving group may be a halogen.

L¹ may be absent, a C₁₋₆ alkylene, a C₂₋₆ alkenylene or a C₂₋₆ alkynylene. Preferably, L¹ is absent or a C₁₋₃ alkylene, more preferably is absent, -CH₂- or -CH₂CH₂-, and most preferably is -CH₂-.

X¹ is preferably O.

L² may be absent, a C₁₋₆ alkylene, a C₂₋₆ alkenylene or a C₂₋₆ alkynylene. Preferably, L² is absent or a C₁₋₃ alkylene, more preferably is absent, -CH₂- or -CH₂CH₂-, and most preferably is absent or -CH₂-.

In one embodiment, R² may be an optionally substituted C₂-C₆ alkenyl or an optionally substituted C₂-C₆ alkynyl. More preferably, R² is a C₂-C₃ alkenyl or an optionally substituted C₂-C₃ alkynyl. Most preferably, R² is -CH₂CHCH₂.

Accordingly, the electrophile may by allyl glycidyl ether (AGE).

In an alternative embodiment, R² may be an optionally substituted 3 membered heterocyclic ring.

Accordingly, R² may be 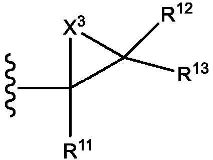
, wherein R¹¹, R¹² and R¹³ are independently H, OR¹⁴, COOR¹⁴, NR¹⁴R¹⁵, a halogen, an optionally substituted C₁₋C₆ alkyl, an optionally substituted C₂-C₆ alkenyl or an optionally substituted C₂-C₆ alkynyl;
X3 is NR¹⁵, O or S; and
R¹⁴ to R¹⁵ are independently H, an optionally substituted C₁₋C₆ alkyl, an optionally substituted C₂-C₆ alkenyl or an optionally substituted C₂-C₆ alkynyl.

Preferably, R¹¹, R¹² and R¹³ are independently H, a C₁-C₃ alkyl, a C₂-C₃ alkenyl or a C₂-C₃ alkynyl. Preferably, R¹¹, R¹² and R¹³ are each H.

X³ may be O.

Accordingly, the electrophile may by diglycidyl ether.

The substrate and the electrophile may be contacted in a weight ratio of between 100:1 and 1:100, between 50:1 and 1:50, between 25:1 and 1:25, more preferably between 20:1 and 1:10, between 15:1 and 1:5 or between 10:1 and 1:1, and most preferably between 8:1 and 2:1, between 6:1 and 3:1 or between 5:1 and 3.5:1.

The substrate and the electrophile may be contacted in a weight ratio sufficient to provide a base matrix comprising between 1 and 500 µg allyl groups or epoxide groups per gram, more preferably between 5 and 250 µg allyl groups or epoxide groups per gram or between 10 and 100 µg allyl groups or epoxide groups per gram, and most preferably between 20 and 70 µg allyl groups or epoxide groups per gram or between 30 and 80 µg allyl groups or epoxide groups per gram.

The substrate and the electrophile may be contacted in a solvent. The solvent may comprise water and/or an alcohol. The alcohol may be ethanol, propan-1-ol and/or propan-2-ol. In some embodiments, the solvent is water.

The substrate and the electrophile may be contacted under alkaline conditions. Accordingly, the substrate and the electrophile may be contacted in the presence of a base. The base may be sodium hydroxide (NaOH). The base may be present at a concentration between 0.001 and 15 M, more preferably between 0.005 and 10 M, between 0.01 and 5 M or between 0.05 and 2 M, and most preferably between 0.1 and 1 M, between 0.2 and 0.75 M or between 0.3 and 0.5 M.

The substrate and the electrophile may be contacted in the presence of an emulsifying agent. The emulsifying agent could be sodium sulphate.

The weight ratio of the substrate to the emulsifying agent may be between 100,000:1 and 1:1,000, between 10,000:1 and 1:500 or between 1,000:1 and 1:100, more preferably is between 500:1 and 1:50, between 100:1 and 1:25, between 50:1 and 1:10 or between 25:1 and 1:1, and most preferably between 10:1 and 2:1 or between 5:1 and 3:1.

The substrate and the electrophile may be contacted in the presence of a reducing agent. The reducing agent may be sodium borohydride.

The weight ratio of the substrate to the reducing agent may be between 100,000:1 and 1:1, between 10,000:1 and 10:1 or between 1,000:1 and 100:1, more preferably is between 900:1 and 150:1, between 800:1 and 200:1, between 700:1 and 250:1 or between 600:1 and 300:1, and most preferably between 500:1 and 350:1 or between 450:1 and 375:1.

The substrate and the electrophile may be contacted at a temperature between 0 and 100°C, more preferably between 10 and 90°C, between 20 and 80°C or between 30 and 70°C, and most preferably between 40 and 60°C or between 45 and 55°C.

The substrate and the electrophile may be contacted for at least 1 minute, at least 30 minutes, at least 1 hour or at least 2 hours, and more preferably for at least 5 hours, at least 10 hours or at least 15 hours. The substrate and the electrophile may be contacted for between 1 minute and 100 hours, between 1 hour and 50 hours or between 2 and 40 hours, more preferably between 5 and 30 hours, between 10 and 25 hours, between 15 and 20 hours or between 17 and 18 hours.

In some embodiments, the method comprises contacting a base matrix comprising an unsaturated hydrocarbon chain with a halogenating matrix.

Prior to contacting the halogenating agent, the base matrix comprising an unsaturated hydrocarbon chain may be washed with a solvent. The solvent may be water and/or an alcohol. The alcohol may be ethanol.

The halogenating agent may be a fluorinating agent, a chlorinating agent or a brominating agent. Preferably, the halogenating agent is a brominating agent.

The halogenating agent may be N-bromosuccinimide, N-chlorosuccinimide, bromine or chlorine. Preferably, the halogenating agent is N-bromosuccinimide.

The weight ratio of the substrate to the halogenating agent may be between 100,000:1 and 1:100 or between 10,000:1 and 1:10, more preferably is between 1,000:1 and 1:1, between 500:1 and 5:1 or between 250:1 and 10:1, and most preferably between 100:1 and 20:1 or between 100:2 and 100:3.

The base matrix comprising an unsaturated hydrocarbon chain may be contacted with the halogenating agent at a temperature between -20 and 100°C, more preferably between 0 and 75°C, between 5 and 50°C or between 10 and 30°C, and most preferably between 15 and 25°C.

The base matrix comprising an unsaturated hydrocarbon chain may be contacted with the halogenating agent in a solvent. The solvent may be water.

The base matrix comprising an unsaturated hydrocarbon chain may be contacted with the halogenating agent under acidic conditions. The base matrix comprising an unsaturated hydrocarbon chain may be contacted at a pH between 1 and 7, more preferably between 2 and 6 or between 3 and 5, and most preferably between 3.5 and 4.5.

The base matrix comprising an unsaturated hydrocarbon chain may be contacted for at least 1 minute, at least 10 minutes, at least 20 minutes or at least 30 minutes, and more preferably for at least 40 minutes, at least 50 minutes or at least 1 hour. The base matrix comprising an unsaturated hydrocarbon chain may be contacted for between 1 minute and 24 hours, between 10 minutes and 12 hours or between 30 minutes and 6 hours, more preferably between 40 minutes and 3 hours, between 50 minutes and 2 hours or between 60 and 90 minutes.

Prior to contacting the aminating agent, the halogenated base matrix may be washed with a solvent. The solvent may be water.

The aminating agent may be NH₂R¹⁶ or 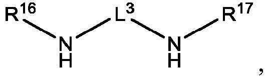 wherein R¹⁶ and R¹⁷ are independently H, an optionally substituted C₁₋₂₄ alkyl, an optionally substituted C₂₋₂₄ alkenyl or an optionally substituted C₂₋₂₄ alkynyl and L³ is an optionally substituted C₁₋₁₂ alkylene, an optionally substituted C₂₋₁₂ alkenylene or an optionally substituted C₂₋₁₂ alkynylene, where the backbone of the alkylene, alkenylene or alkynlene is optionally interrupted by one or more heteroatoms.

Preferably, R¹⁶ and R¹⁷ are both H.

Preferably, L³ is a C₁₋₆ alkylene, a C₂₋₆ alkenylene or a C₂₋₆ alkynylene. More preferably, L³ is a C₁₋₃ alkylene, most preferably is-CH₂- or -CH₂CH₂-.

Preferably, the aminating agent is ammonia.

The halogenated base matrix and the aminating agent may be contacted in a solvent. The solvent may be water.

The halogenated base matrix and the aminating agent may be contacted in a weight ratio of between 100:1 and 1:100, between 50:1 and 1:50, between 25:1 and 1:25, more preferably between 20:1 and 1:20, between 15:1 and 1:10 or between 10:1 and 1:5, and most preferably between 8:1 and 1:2, between 5:1 and 1:1 or between 3:1 and 2:1.

The halogenated base matrix and the aminating agent may be contacted at a temperature between 0 and 100°C, more preferably between 10 and 90°C, between 20 and 80°C or between 30 and 75°C, and most preferably between 45 and 70°C or between 55 and 65°C.

The halogenated base matrix and the aminating agent may be contacted for at least 1 minute, at least 30 minutes, at least 1 hour or at least 2 hours, and more preferably for at least 5 hours, at least 10 hours, at least 15 hours or at least 20 hours. The halogenated base matrix and the aminating agent may be contacted for between 1 minute and 100 hours, between 1 hour and 50 hours or between 2 and 45 hours, more preferably between 5 and 40 hours, between 10 and 35 hours, between 15 and 30 hours or between 20 and 24 hours.

Prior to contacting the heteroaromatic compound, the aminated base matrix may be washed with a solvent. The solvent may be water and/or a pH neutral solution. The aminated base matrix is preferably washed with water and then a pH neutral solution. The pH neutral solution may be an aqueous solution. The pH neutral solution may comprise alkali metal ions, alkaline earth metal ions, phosphate ions, sulphate ions and/or halogen ions. The pH neutral solution may comprise sodium phosphate, potassium phosphate and/or sodium chloride.

The heteroaromatic compound is a heteroaromatic ring which is substituted with at least two halogens, and optionally one or more further substituents. The heteroaromatic ring may be a 5 to 10 membered heteroaromatic ring, more preferably a 5 or 6 membered heteroaromatic ring, and most preferably a 6 membered heteroaromatic ring.

The heteroaromatic ring may be a pyridine ring, a pyridazine ring, a pyrimidine ring, a pyrazine ring, a 1,2,4-triazine ring or a 1,3,5-triazine ring. Preferably, the heteroaromatic group comprises a 1,2,4-triazine ring or a 1,3,5-triazine ring and most preferably a 1,3,5-triazine ring.

The heteroaromatic ring is substituted with at least two halogen atoms, and may be substituted with 2, 3, 4 or 5 halogen atoms. Preferably, the heteroaromatic ring is substituted with 2 or 3 halogen atoms, most preferably 3 halogen atoms.

The or each halogen may be fluorine, chlorine, bromine or iodine. Preferably, the or each halogen is chlorine or bromine. In one embodiment, each halogen is chlorine.

The heteroaromatic ring may be substituted with one or more further substituents. The or each further substituent may be independently selected from the group consisting of OH, SH, COOH, NH₂, optionally substituted C₁-C₂₄ alkyl, optionally substituted C₂-C₂₄ alkenyl and optionally substituted C₂-C₂₄ alkynyl.

The heteroaromatic compound may be dichloro-triazine or cyanuric chloride, and is preferably cyanuric chloride.

The aminated base matrix and the heteroaromatic compound may be contacted in a solvent. The solvent may be water and/or acetone.

The aminated base matrix and the heteroaromatic compound may be contacted in the presence of a buffer. The buffer may be potassium phosphate. The buffer may be present at a concentration of between 0.01 and 100 M, more preferably between 0.05 and 10 M or between 0.1 and 5 M, and most preferably between 0.5 and 2 M or between 0.75 and 1.5 M.

The weight ratio of the aminated base matrix to the heteroaromatic compound may be between 1,000:1 and 1:50 or between 500:1 and 1:25, more preferably is between 200:1 and 1:10, between 100:1 and 1:1, between 75:1 and 10:1 or between 50:1 and 20:1, and most preferably between 40:1 and 25:1 or between 35:1 and 30:1.

The aminated base matrix and the heteroaromatic compound may be contacted at a temperature between -50 and 50°C, more preferably between -30 and 30°C, between - 20 and 20°C or between -10 and 10°C, and most preferably between -5 and 7°C, between 0 and 5°C or between 1 and 3°C.

The aminated base matrix and the heteroaromatic compound may be contacted for at least 1 minute, at least 10 minutes, at least 20 minutes or at least 30 minutes, and more preferably for at least 40 minutes, at least 50 minutes or at least 1 hour. The aminated base matrix and the heteroaromatic compound may be contacted for between 1 minute and 24 hours, between 10 minutes and 12 hours or between 30 minutes and 6 hours, more preferably between 40 minutes and 3 hours, between 50 minutes and 2 hours or between 60 and 90 minutes.

The activated substrate may be washed with a solvent. The solvent may be water and/or acetone. The activated substrate is preferably washed with a water and acetone solution and then water.

In accordance with a second aspect, there is provided the activated substrate obtained or obtainable by the method of the first aspect.

In accordance with a third aspect, there is provided an activated substrate of formula (II): 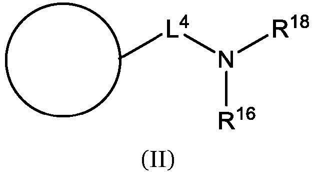
, wherein L⁴ is an optionally substituted C₁₋₂₄ alkylene, an optionally substituted C₂₋₂₄ alkenylene or an optionally substituted C₂₋₂₄ alkynylene, where the backbone of the alkylene, alkenylene or alkynlene is optionally interrupted by one or more heteroatoms; R¹⁶ is H, an optionally substituted C₁₋₂₄ alkyl, an optionally substituted C₂₋₂₄ alkenyl or an optionally substituted C₂₋₂₄ alkynyl; and
R¹⁸ is a 5 to 12 membered heteroaryl group substituted with at least one halogen, and optionally substituted with one or more further substituents.

It may be appreciated that the circle in formula (II) represents a substrate. The substrate may be as defined in relation to the first aspect.

L⁴ may be an optionally substituted C₃₋₁₂ alkylene, an optionally substituted C₃₋₁₂ alkenylene or an optionally substituted C₃₋₁₂ alkynylene, where the backbone of the alkylene, alkenylene or alkynlene is optionally interrupted by one or more heteroatoms. Preferably, L⁴ is an optionally substituted C₅₋₁₀ alkylene, an optionally substituted C₅₋₁₀ alkenylene or an optionally substituted C₅₋₁₀ alkynylene, where the backbone of the alkylene, alkenylene or alkynlene is optionally interrupted by one or more heteroatoms. More preferably, L⁴ is an optionally substituted C₆₋₈ alkylene, an optionally substituted C₆₋₈ alkenylene or an optionally substituted C₆₋₈ alkynylene, where the backbone of the alkylene, alkenylene or alkynlene is optionally interrupted by one or more heteroatoms. Most preferably, L⁴ is an optionally substituted C₇ alkylene, an optionally substituted C₇ alkenylene or an optionally substituted C₇ alkynylene, where the backbone of the alkylene, alkenylene or alkynlene is optionally interrupted by one or more heteroatoms. The alkylene, alkenylene or alkynlene may be substituted with one or more hydroxyl groups. The alkylene, alkenylene or alkynlene may be substituted with between 1 and 5 hydroxyl groups, between 1 and 4 hydroxyl groups or between 2 and 3 hydroxyl groups. Preferably the alkylene, alkenylene or alkynlene is substituted with two hydroxyl groups.

Preferably, the backbone of the alkylene, alkenylene or alkynlene is interrupted with at least one heteroatom. The backbone of the alkylene, alkenylene or alkynlene may be interrupted with between 1 and 5 heteroatoms, between 1 and 4 heteroatoms or between 2 and 3 heteroatoms. Preferably, the backbone of the alkylene, alkenylene or alkynlene is interrupted with two heteroatoms. The one or more heteroatoms may each be O, NR¹¹ or S, wherein R¹¹ is H, an optionally substituted C₁₋C₆ alkyl, an optionally substituted C₂-C₆ alkenyl or an optionally substituted C₂-C₆ alkynyl. Preferably, the or each heteroatom is oxygen. Preferably, the backbone is interrupted with at least two oxygen atoms.

Accordingly, L⁴ may be 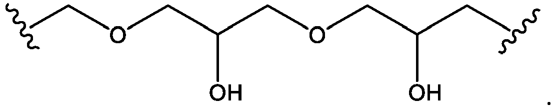 R¹⁶ may be H, an optionally substituted C₁₋₁₂ alkyl, an optionally substituted C₂₋₁₂ alkenyl or an optionally substituted C₂₋₁₂ alkynyl. Preferably, R¹⁶ is H, an optionally substituted C₁₋₆ alkyl, an optionally substituted C₂₋₆ alkenyl or an optionally substituted C₂₋₆ alkynyl. More preferably, R¹⁶ is H, an optionally substituted C₁₋₃ alkyl, an optionally substituted C₂₋₃ alkenyl or an optionally substituted C₂₋₃ alkynyl. Most preferably, R¹⁶ is H.

R¹⁸ may be a 5 to 10 membered heteroaryl group substituted with at least one halogen, and optionally substituted with further substituents, more preferably a 5 or 6 membered heteroaryl group substituted with at least one halogen, and optionally substituted with further substituents, and most preferably a 6 membered heteroaryl group substituted with at least one halogen, and optionally substituted with further substituents.

The heteroaryl group which is substituted with at least one halogen, and optionally one or more further substituents, may be a pyridinyl, a pyridazinyl, a pyrimidinyl, a pyrazinyl, a 1,2,4-triazinyl or a 1,3,5-triazinyl. Preferably, the heteroaryl group is a 1,2,4-triazinyl or a 1,3,5-triazinyl substituted with at least one halogen, and optionally one or more further substituents and most preferably 1,3,5-triazinyl substituted with at least one halogen, and optionally one or more further substituents.

The heteroaryl group is substituted with at least one halogen atom, and may be substituted with 1, 2, 3 or 4 halogen atoms. Preferably, the heteroaryl group is substituted with 1 or 2 halogen atoms, most preferably 2 halogen atoms.

The or each halogen may be fluorine, chlorine, bromine or iodine. Preferably, the or each halogen is chlorine or bromine. In one embodiment, each halogen is chlorine.

The heteroaryl group may be substituted with one or more further substituents. The one or more further substituents may be OH, SH, COOH, NH₂, optionally substituted C₁-C₂₄ alkyl, optionally substituted C₂-C₂₄ alkenyl or optionally substituted C₂-C₂₄ alkynyl.

Accordingly, R¹⁸ may be 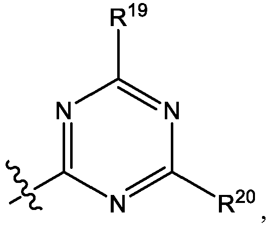 wherein R¹⁹ is a halogen and R²⁰ is a halogen, H, OH, SH, COOH, NH₂, optionally substituted C₁-C₂₄ alkyl, optionally substituted C₂-C₂₄ alkenyl or optionally substituted C₂-C₂₄ alkynyl. Preferably, R²⁰ is a halogen or H.

Accordingly, in a preferring embodiment, the compound of formula (II) is a compound of formula (IIa):

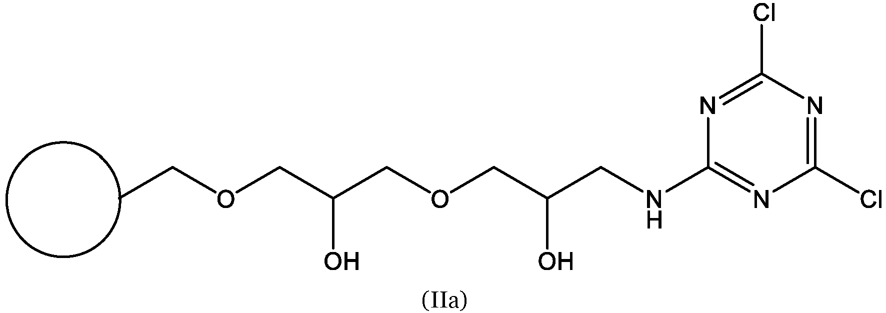

In accordance with a fourth aspect, there is provided a method of producing a scaffold for isolation of a biomolecule, the method comprising:
- conducting the method of the first aspect to produce an activated substrate, or providing the activated substrate of the second or third aspect; and
- contacting the activated substrate with a molecule comprising a ligand specific for a biomolecule to provide a scaffold for isolation of the biomolecule.

It will be understood that the ligand may be selected according to the biomolecule to be isolated.

Advantageously, the method provides an alkaline-stable structure which can be used as an affinity medium or ligand for bioprocessing, where sanitisation with caustic solutions is highly desirable.

The biomolecule may be selected from the group consisting of an amino acid, a peptide, an affimer, a protein, an enzyme, a glycoprotein, a lipopolysaccharide, an antibody or a fragment thereof, a nucleic acid, an organic polymer, a virus, a bacterium, a cell, and a cell-related structure. The antibody may be an isoagglutinin. The virus may be adenoassociated virus (AAV) or Lentivirus. The cell may be an animal cell.

The molecule for forming the ligand attached to the activated substrate may be a compound of formula (IV):

X⁴-L⁵-X⁵ (IV)

, wherein X⁴ is NH₂, SH or OH;
L⁵ is absent or is an optionally substituted C₁₋₃₀ alkylene, an optionally substituted C₂₋₃₀ alkenylene or an optionally substituted C₂₋₃₀ alkynylene where the backbone of the alkylene, alkenylene or alkynlene is optionally interrupted by one or more heteroatoms, or an optionally substituted C₆₋₂₀ arylene; and
X⁵ is a ligand specific for a biomolecule.

X⁴ may be NH₂ or SH. Preferably, X⁴ is NH₂.

L⁵ may be an optionally substituted C₃₋₂₀ alkylene, an optionally substituted C₃₋₂₀ alkenylene or an optionally substituted C₃₋₂₀ alkynylene, where the backbone of the alkylene, alkenylene or alkynlene is optionally interrupted by one or more heteroatoms. More preferably, L⁵ is an optionally substituted C₅₋₁₅ alkylene, an optionally substituted C₅₋₁₅ alkenylene or an optionally substituted C₅₋₁₅ alkynylene, where the backbone of the alkylene, alkenylene or alkynlene is optionally interrupted by one or more heteroatoms. Most preferably, L⁵ is an optionally substituted C₈₋₁₂ alkylene, an optionally substituted C₈₋₁₂ alkenylene or an optionally substituted C₈₋₁₂ alkynylene, where the backbone of the alkylene, alkenylene or alkynlene is optionally interrupted by one or more heteroatoms.

L⁵ may be an alkylene, an alkenylene or an alkynylene where the alkylene, alkenylene or alkynylene is substituted with one or more oxo groups. Preferably, the alkylene, alkenylene or alkynylene is substituted with an oxo group.

L⁵ may be an alkylene, an alkenylene or an alkynylene wherein the backbone of the alkylene, alkenylene or alkynylene is interrupted by one or more heteroatoms selected from NR²⁸, O or S, wherein R²⁸ is H, an optionally substituted C₁₋₆ alkyl, an optionally substituted C₂₋₆ alkenyl or an optionally substituted C₂₋₆ alkenyl. More preferably, L⁵ is an alkylene, an alkenylene or an alkynylene wherein the backbone of the alkylene, alkenylene or alkynylene is interrupted by an NH group.

The ligand may comprise an optionally derivatized saccharide molecule, an optionally derivatized amino acid, an optionally derivatized peptide, an optionally derivatized affimer or an optionally derivatized protein. The optionally derivatized saccharide molecule may be an optionally derivatives polysaccharide molecule.

The ligand may be derivatised with one or more functional groups. The one or more functional groups may replace a hydrogen or hydroxyl group in the ligand. In embodiments where the ligand is a polysaccharide, one or more of the saccharide monomers may be derivatized. The one or more functional groups may be an optionally substituted C₁₋₆ alkyl, an optionally substituted C₂₋₆ alkenyl, an optionally substituted C₂₋₆ alkenyl, OR²⁶, SR²⁶, C(O)R²⁶, NR²⁶R²⁷, NR²⁶C(O)R²⁷ or SO₃R²⁶, wherein R²⁶ and R²⁷ are independently H, an optionally substituted C₁₋₆ alkyl, an optionally substituted C₂₋₆ alkenyl or an optionally substituted C₂₋₆ alkenyl.

The saccharide molecule may comprise between 1 and 20 saccharide monomers, more preferably between 2 and 10 saccharide monomers or between 2 and 8 saccharide monomers, and most preferably between 3 and 6 saccharide monomers.

The or each optionally derivatized saccharide monomer may be selected from the group consisting of optionally derivatized glucose; optionally derivatized glucosamine; optionally derivatized galactose; optionally derivatized fructose; and optionally derivatized xylose, or a stereoisomer thereof.

In some embodiments, X⁵ is 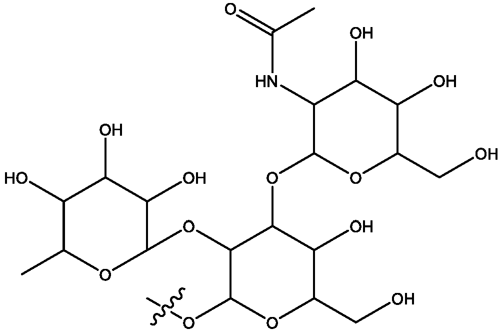 or

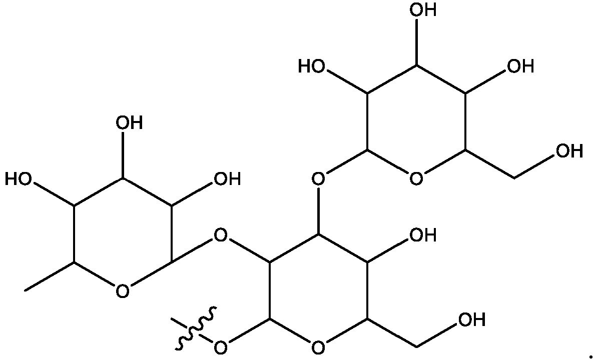

More preferably, X⁵ is 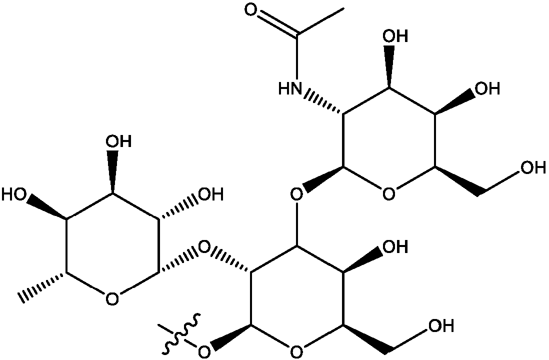 or

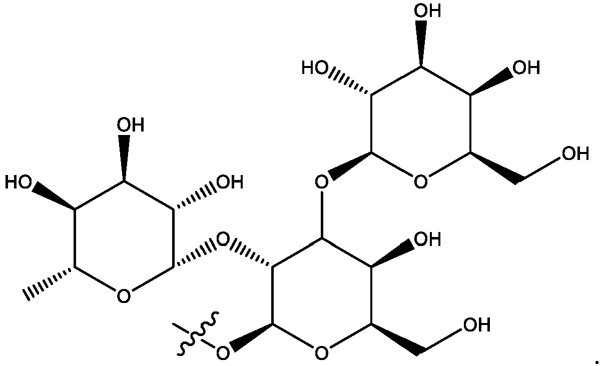

The compound of Formula (IV) may be an antigen-A trisaccharide ligand, which has Formula (IVa):

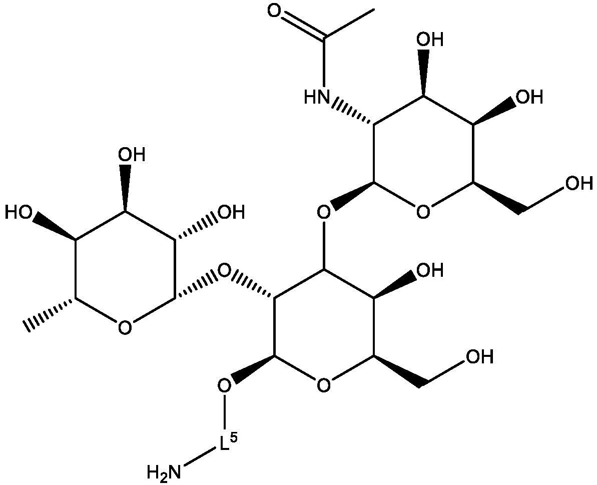

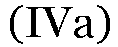

The compound of Formula (IV) may be an antigen-B trisaccharide ligand, which has Formula (IVb):

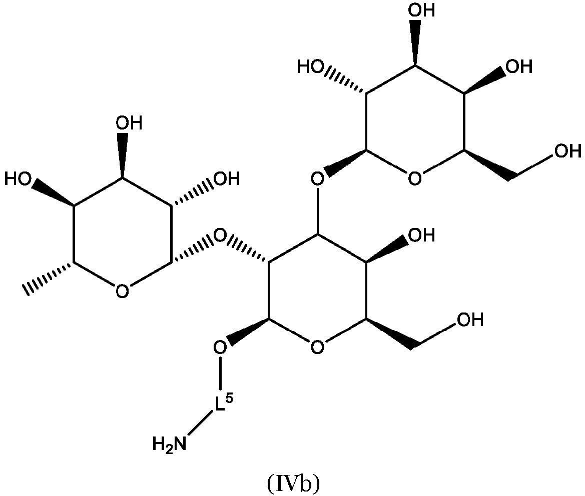

The ligand may have an affinity for isoagglutinins.

The ligand may, in use, be cationic, preferably protonated.

An exemplary ligand which may be cationic in use is a ligand formed from a compound of formula (IVc):

H₂N-L⁵-N(Ak-NH₂)₂ (IVc)

wherein Ak in each occurrence is a C₁₋₁₂ alkylene where, in the case of a C₂₋₁₂ alkylene, the backbone of the alkylene is optionally interrupted by one or more heteroatoms, e.g one or more O atoms.

A preferred compound of formula (IVc) is formula (IVd):

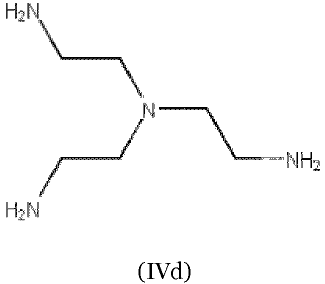

The ligand formed from the compound of formula (IVd) may have an affinity to lipopolysaccharide and albumin.

X⁵ of formula (IV) may be a boronate group. A ligand comprising a boronate group may be formed from a compound of formula (IVe):

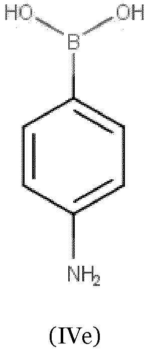

The ligand may have an affinity to glycosylated proteins.

X⁵ of the compound of Formula (IV) may be a naphthol ligand. An exemplary compound of formula (IV) comprising a naphthol ligand has Formula (IVf):

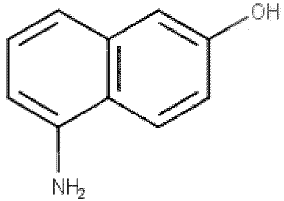

The ligand may have an affinity to insulin.

The method may comprise contacting the activated substrate and the molecule comprising the ligand in a solvent. The solvent may be water.

The activated substrate and the molecule comprising the ligand may be contacted in a weight ratio of between 1,000,000:1 and 1:1 or between 100,000:1 and 10:1, more preferably between 10,000:1 and 25:1 or between 5,000:1 and 50:1, and most preferably between 2,000:1 and 100:1, between 1,500:1 and 200:1 or between 1,000:1 and 1,000:3.

Alternatively, or additionally, the ligand may be present at a concentration of between 1 µg/ml and 750 mg/ml, between 10 µg/ml and 500 mg/ml or between 50 µg/ml and 250 mg/ml, more preferably between 100 µg/ml and 100 mg/ml, between 250 µg/ml and 50 mg/ml, between 500 µg/ml and 10 mg/ml or between 750 µg/ml and 5 mg/ml, most preferably between 1 and 3 mg/ml.

Alternatively or additionally, the ligand may be present at a concentration at least 40 µmol per gram of scaffold, optionally a concentration of at least 50 µmol/g, optionally up to 300 or 200 µmol / g.

The activated substrate and the molecule comprising the ligand may be contacted under alkaline conditions. The activated substrate and the molecule comprising the ligand may be contacted in solution at a pH between 6 and 14 at 20°C, more preferably at a pH between 7 and 13 at 20°C, and most preferably at a pH of between 8 and 12 at 20°C.

The activated substrate and the molecule comprising the ligand may be contacted at a temperature between -20 and 100°C, more preferably between 0 and 75°C, between 5 and 50°C or between 10 and 30°C, and most preferably between 15 and 25°C.

The activated substrate and the ligand molecule comprising the may be contacted for at least 1 minute, at least 10 minutes, at least 20 minutes or at least 30 minutes, and more preferably at least 1 hour, at least 2 hours, at least 3 hours or at least 4 hour. The activated substrate and the molecule comprising the ligand may be contacted for between 1 minute and 48 hours, between 30 minutes and 24 hours or between 1 and 12 hours, more preferably between 2 and 10 hours, between 3 and 8 hours or between 4 to 6 hours.

The method may subsequently comprise contacting the scaffold with an alcohol, a hydroxide, ammonia, an amine or a thiol. The alcohol, hydroxide, ammonia, amine or thiol may be HOR²⁴, -OH, HNR²⁴R²⁵ or HSR²⁴, wherein R²⁴ and R²⁵ are independently H, optionally substituted C₁-C₁₂ alkyl, optionally substituted C₂-C₁₂ alkenyl or optionally substituted C₂-C₁₂ alkynyl. More preferably, R²⁴ and R²⁵ are independently H, optionally substituted C₁-C₆ alkyl, optionally substituted C₂-C₆ alkenyl or optionally substituted C₂-C₆ alkynyl. Most preferably, R²⁴ and R²⁵ are independently H, optionally substituted C₁-C₃ alkyl, optionally substituted C₂-C₃ alkenyl or optionally substituted C₂-C₃ alkynyl. The alkyl, alkenyl or alkynyl may be optionally substituted with -OH, NH₂ or SH.

The alcohol, hydroxide, ammonia, amine or thiol may be selected from the group consisting of 2-aminoethanol, methylamine, ammonia, sodium hydroxide, glycine, alanine, dimethylamine, tris(hydroxymethyl)aminomethane or 2-mercaptoethanol. Advantageously, this step removes residual halogen sites after ligand coupling.

The scaffold and the alcohol, hydroxide, ammonia, amine or thiol may be contacted in a weight ratio of between 1:10 and 1,000:1 or between 1:1 and 500:1, more preferably between 2:1 and 250:1 or between 4:1 and 100:1, and most preferably between 10:1 and 50:1, between 25:2 and 25:1 or between 100:6 and 100:5.

The scaffold may be washed with a solvent. The solvent may be water.

In accordance with a fifth aspect, there is provided the scaffold for isolation of a biomolecule obtained or obtainable by the method of the fourth aspect.

In accordance with a sixth aspect, there is provided a scaffold for isolation of a biomolecule, wherein the scaffold is represented by formula (III):

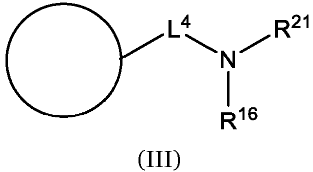

, wherein L⁴ and R¹⁶ are as defined in relation to the third aspect; and R²¹ is a 5 to 12 membered heteroaryl group substituted with at least one group comprising a ligand specific for a biomolecule, and optionally substituted with one or more further substituents.

The group comprising the ligand and biomolecule may be as defined in relation to the fourth aspect.

In particular, the group comprising the ligand may have formula -L⁶-L⁵-X⁵, wherein L⁵ and X⁵ are as defined in the fourth aspect and L⁶ is O, S or NH.

R²¹ may be a 5 to 10 membered heteroaryl group substituted with at least one group comprising the ligand specific for a biomolecule, and optionally substituted with one or more further substituents, more preferably a 5 or 6 membered heteroaryl group substituted with at least one group comprising the ligand specific for a biomolecule, and optionally substituted with one or more further substituents, and most preferably a 6 membered heteroaryl group substituted with at least one group comprising the ligand specific for a biomolecule, and optionally substituted with one or more further substituents.

The heteroaryl group which is substituted with at least one group comprising the ligand specific for a biomolecule, and optionally substituted with one or more further substituents, may be a pyridinyl, a pyridazinyl, a pyrimidinyl, a pyrazinyl, a 1,2,4-triazinyl or a 1,3,5-triazinyl. Preferably, the heteroaryl group is a 1,2,4-triazinyl or a 1,3,5-triazinyl substituted with at least one group comprising the ligand specific for a biomolecule, and optionally substituted with one or more further substituents and most preferably a 1,3,5-triazinyl substituted with at least one group comprising the ligand specific for a biomolecule, and optionally substituted with one or more further substituents.

The heteroaryl group is substituted with at least one group comprising the ligand, and may be substituted with 1, 2, 3 or 4 groups comprising the ligand. Preferably, the heteroaryl group is substituted with 1 or 2 group comprising the ligand, most preferably 2 group comprising the ligand.

The heteroaryl group may be substituted with one or more further substituents. The one or more further substituents may be a halogen, OR²⁴, SR²⁴, COOR²⁴, NR²⁴R²⁵, optionally substituted C₁-C₂₄ alkyl, optionally substituted C₂-C₂₄ alkenyl or optionally substituted C₂-C₂₄ alkynyl, wherein R²⁴ and R²⁵ are independently H optionally substituted C₁-C₂₄ alkyl, optionally substituted C₂-C₂₄ alkenyl or optionally substituted C₂-C₂₄ alkynyl.

Accordingly, R²¹ may be 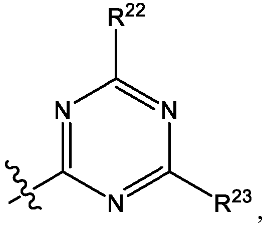 wherein R²² is a group comprising a ligand specific for a biomolecule and R²³ is a group comprising a ligand specific for a biomolecule, a halogen, H, OR²⁴, COOR²⁴, NR²⁴R²⁵, SR²⁴, optionally substituted C₁-C₂₄ alkyl, optionally substituted C₂-C₂₄ alkenyl or optionally substituted C₂-C₂₄ alkynyl, wherein R²⁴ and R²⁵ are independently H optionally substituted C₁-C₂₄ alkyl, optionally substituted C₂-C₂₄ alkenyl or optionally substituted C₂-C₂₄ alkynyl. Preferably, R²⁴ and R²⁵ are independently H, optionally substituted C₁-C₁₂ alkyl, optionally substituted C₂-C₁₂ alkenyl or optionally substituted C₂-C₁₂ alkynyl. More preferably, R²⁴ and R²⁵ are independently H, optionally substituted C₁-C₆ alkyl, optionally substituted C₂-C₆ alkenyl or optionally substituted C₂-C₆ alkynyl. Most preferably, R²⁴ and R²⁵ are independently H, optionally substituted C₁-C₃ alkyl, optionally substituted C₂-C3₆ alkenyl or optionally substituted C₂-C₃ alkynyl. The alkyl, alkenyl or alkynyl may be optionally substituted with -OH, NH₂ or SH.

In a most preferred embodiment, the scaffold is represented by formula (IIIa):

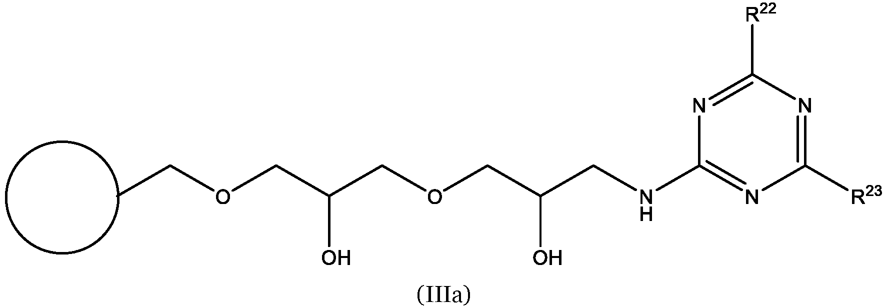

R²² may be 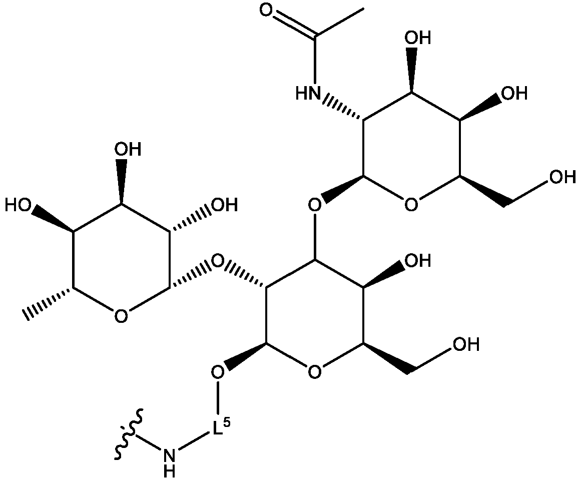 or

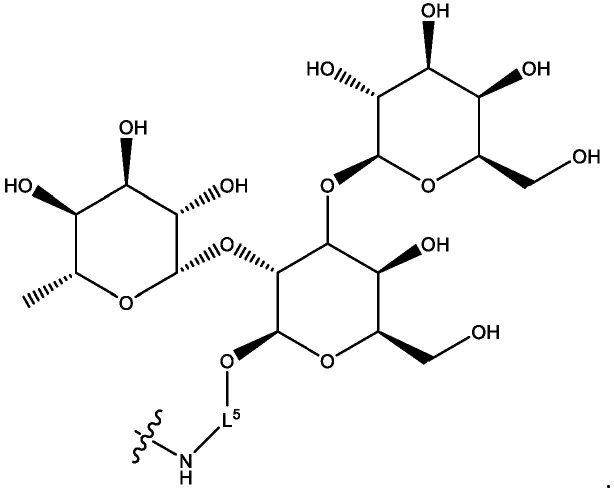

Preferably, R²³ is a group comprising a ligand specific for a biomolecule. Preferably, R²³ is the same as R²².

In accordance with a seventh aspect, there is provided use of the scaffold of the fifth or sixth aspect to isolate a biomolecule.

In accordance with an eighth aspect, there is provided a method of isolating a biomolecule on a scaffold, the method comprising contacting a scaffold with a biomolecule, wherein the scaffold is as defined in either the fifth or sixth aspect.

It may be appreciated that the use of the seventh aspect is in affinity chromatography. The method of the eighth aspect is preferably a method of conducting affinity chromatography.

The biomolecule may be as defined in relation to the fourth aspect.

The method may comprise contacting the scaffold for isolation of a biomolecule with a solution comprising the biomolecule.

The biomolecule may be present in human intravenous immunoglobulin (IVIG) intermediate product. Accordingly, the method may comprise contacting the scaffold for isolation of a biomolecule with human IVIG intermediate product.

In accordance with a ninth aspect, there is provided a method of cleaning a scaffold, the method comprising contacting a scaffold with a caustic substance, wherein the scaffold is as defined in either the fifth or sixth aspect.

Advantageously, the scaffold may be cleaned to avoid contamination from bacteria, viruses, and endotoxin without loss of performance.

The caustic substance may be or comprise an alkaline solution.

The alkaline solution may have a pH of at least 7.5, at least 8, at least 9, at least 10, at least 11, at least 12 or at least 13 at 20°C. The alkaline solution may have a pH of between 8 and 14.5, between 9 and 14, between 10 and 13.75, between 11 and 13.5, between 12 and 13.25 or between 12.5 and 13 at 20°C.

The alkaline solution may comprise an alkali metal hydroxide or an alkaline earth metal hydroxide. Preferably, the alkaline solution comprises an alkali metal hydroxide. The alkaline solution may comprise lithium hydroxide, sodium hydroxide or potassium hydroxide. In some embodiments, the alkaline solution comprises sodium hydroxide. The alkaline solution may comprise the alkali metal hydroxide or the alkaline earth metal hydroxide at a concentration of between 0.01 and 10 M, between 0.05 and 5 M, between 0.1 and 2.5 M, between 0.2 and 1 M, between 0.3 and 0.75 M or between, between 0.4 and 0.6 M.

The method of the ninth aspect may be performed subsequent to the method of the eighth aspect. The scaffold may then be used in a further method of isolating a biomolecule. Accordingly, the method of the eighth aspect may be repeated after the method of the ninth aspect.

The methods of the eighth and ninth aspect may be cycled, one after the other. The methods of the eighth and ninth aspect may be repeated at least 2 times, at least 3 times at least 4 times or at least 5 times, more preferably at least 10 times, at least 20 times or at least 30 times, and most preferably at least 40 or 50 times.

All of the features described herein (including accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying drawings, in which:-
**Figure 1** shows a synthesis route and structure of dihalogentriazine activated base matrix represented by dichlorotriazine;
**Figure 2** shows an agglutination assay plate for A-antigen adsorbent. Two replicate load samples were applied to the top two rows and four non-bound samples (labelled NB rep. 1 and 2), recovered from the A-isoagglutinin column in two separate experiments (Exp. 1 and 2), are shown in the bottom four rows. The dilutions of the loads are indicated above the top row, while the figures below the bottom row indicate the dilutions applied to the non-bound samples;
**Figure 3** shows as agglutination assay plate for B-antigen adsorbent. Two replicate load samples were applied to the top two rows and two replicate non-bound samples (labelled NB Rep. 1 and 2), recovered from the B-isoagglutinin column, are shown in the bottom two rows. The dilutions of the loads are indicated above the top row, while the figures below the bottom row indicate the dilutions applied to the non-bound samples;
**Figure 4** is a graph of A-antigen trisaccharide ligand concentration vs. time in the presence of dihalogentriazine activated base matrices having differing activation densities;
**Figure 5** is a graph of BSA binding capacities across 21 caustic cleaning cycles for a dihalogentriazine scaffold and comparative scaffolds having a Tren ligand;
**Figure 6** is a graph of HSA binding capacities across 21 caustic cleaning cycles for a dihalogentriazine scaffold and comparative scaffolds having a Blue ligand;
**Figure 7** is a graph of Blue ligand leachate over time for a dihalogentriazine scaffold and a comparative scaffold in NaOH; and
**Figure 8** is a graph of binding capacity over time for a dihalogentriazine scaffold and a comparative scaffold having a Blue ligand in NaOH;

### Examples

### EXAMPLE 1

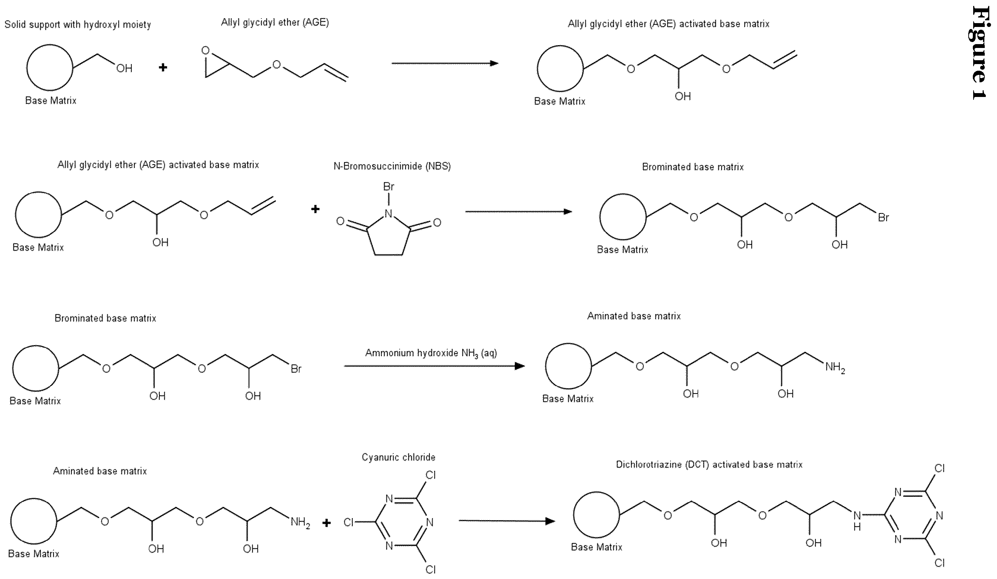

A synthetic route for producing a dihalogentriazine scaffold is shown in Figure 1. The steps are described in more detail below.

### Solid phase activation of a solid support

### 1.1 Allyl activation

Approximately 1 kg of a solid support, such as beaded agarose, is slurried in water with up to 380 g, optionally approximately 250g of sodium sulphate, 10 M NaOH, and 2.5 g of sodium borohydride. The slurry is heated to 45 to 55 °C before adding allyl glycidyl ether (up to 1800 mL, optionally 250 mL). The slurry is left to react for no more than 18 hours, after which the drained gel is washed with ethanol and water. The resulting material is an activated base matrix that contains up to about 200 µmol allyl groups per g adsorbent.

### 1.2 Bromination of allyl-activated base matrix

The allyl-activated base matrix is slurried at room temperature with an acidic solution of pH around 4, followed by the addition of N-bromosuccinimide, and incubation for at least one hour. The resulting brominated base matrix is then washed with water and left to settle.

### 1.3 Amination of brominated base matrix

The brominated base matrix is resuspended in water followed by the addition of 600 mL of an ammonia solution, and heating to no more than 65 °C under stirring for less than 24 hours. At the end of the reaction period, the reaction is drained and washed with water, then a pH-neutral solution, followed by settling.

### 1.4 Dichlorotriazine (DCT) activation of the aminated base matrix

The aminated base matrix resulting from the previous step is then slurried in an aqueous solution of potassium phosphate (1M), followed by settling and re-suspension in the same solution, with the addition of 160 mL of acetone, under stirring at around 2 °C. Approximately 1.4 molar equivalents of cyanuric chloride with respect to the precursor activation density dissolved in acetone is added to the slurried aminated base matrix, followed by incubation under refrigeration for approximately one hour, after which it is drained, washed with aqueous acetone solutions of decreasing concentration, then a final wash in water, after which the slurry is settled under gravity. The final product of this reaction is the DCT-activated base matrix.

### EXAMPLE 2

### Functionalisation of an activated solid support

### 2.1 Coupling an antigen-A trisaccharide ligand to DCT-activated base matrix

A DCT-activated base matrix is suspended in water and set to stir at room temperature. A solution of A-antigen trisaccharide ligand comprising a flexible linker in water is added to the DCT gel slurry maintained under high pH at room temperature for 4 to 6 hours. The A-antigen trisaccharide ligand comprising the flexible linker is a compound of formula (IVa), as described herein. After reaction, the derivatised base matrix is blocked using mercaptoethanol and thoroughly washed with water before being allowed to settle under gravity.

This reaction results in a chromatographic scaffold material containing an affinity ligand which binds A-isoagglutinins (Scaffold Example 1).

### 2.2 Coupling antigen-B trisaccharide ligand to DCT-activated base matrix

A DCT-activated base matrix is suspended in water and set to stir at room temperature. A solution of B-antigen trisaccharide ligand comprising a flexible linker in water is added to the DCT gel slurry maintained under high pH conditions at room temperature for 4 to 6 hours. The B-antigen trisaccharide ligand comprising the flexible linker is a compound of formula (IVb), as described herein. After reaction, the derivatised base matrix is blocked using mercaptoethanol, thoroughly washed with water then settled under gravity.

This reaction results in a chromatographic scaffold material containing an affinity ligand which binds B-isoagglutinins (Scaffold Example 2).

### EXAMPLE 3

### 3.1 Removal of A-isoagglutinin from human IVIG intermediate product feedstock using a product of this invention

A column containing the antigen A ligand (Scaffold Example 1) is packed, followed by the application of an IVIG solution containing isoagglutinins at a flow rate of 1 mL/min. The flow-through from the loading process is collected. The load and flow-through samples are analysed for of A-isoagglutinins using a standard agglutination assay.

### 3.2 Agglutination assay

### 3.2.1 Preparation of red blood cells

Human red blood cells are washed with PBS buffer by adding 2mL red blood cells into a 5 mL centrifuge tube and centrifuging at 1400 rpm for 3 minutes. The supernatant from the centrifuged cells is removed before adding PBS buffer to the cell pellet to make the suspension up to a 2 mL volume and the cells mixed by inverting the tube. This PBS wash is repeated three times.

Freeze dried papain is reconstituted with PBS buffer (2 mL) before adding 200 µL to the cells after centrifugation and removal of the supernatant. These suspensions are then made up to 2 mL volume with PBS buffer before incubation with mixing for 10 minutes at 37 °C.

At the end of this incubation period, the suspension is centrifuged for 3 minutes at 1400 rpm. The supernatant is removed from the centrifuged cells, then PBS buffer added to make the suspension up to a 2 mL volume. The cells are mixed by inverting the tube. This PBS wash is repeated three times.

The spun cells are mixed with inversion made up to 2 mL volume with 2 mg/mL BSA solution.

### 3.2.2 Performing the agglutination test

A sample of the feedstock from step 3.1 is diluted 1/2, 1/4, 1/6, 1/8, 1/10, 1/12, 1/14 and 1/16 with a solution of 2 mg/mL BSA solution.

A sample of the non-bound from step 3.1 is diluted 1/2, 1/3, 1/4, 1/5, 1/6, 1/7, 1/8 and 1/9 with a solution of 2 mg/mL BSA solution.

The feedstock and non-bound dilutions described above (20 µL) are transferred to a 96 well v-bottomed plate along with the blank 2mg/mL BSA solution.

The red blood cells prepared in stage 3.2.1 are mixed by inversion before transferring to a pipette reservoir. The blood cell suspension (20 µL) is transferred to the plate using a multichannel pipette. The plate is agitated to mix the solutions for 30 seconds before centrifuging the plate at 1400 rpm for 3 minutes.

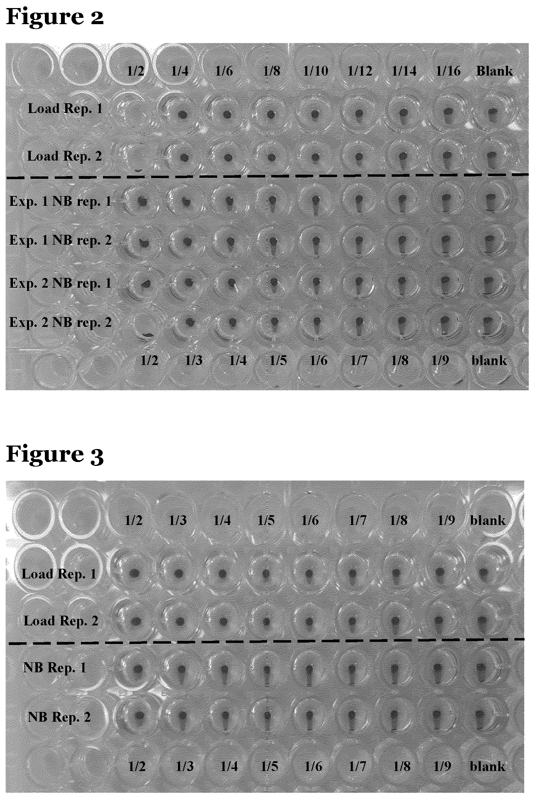

The plate is placed onto a stand at a 70° angle for 15 minutes. Each well of the plate is studied to determine the level of clearance of isoagglutinin from the red blood cells. This is achieved by assessing the sample dilution required to prevent agglutination, indicated by streaming of the red blood cells in the plate wells (Figure 2).

As shown in Figure 2, a dilution of greater than 1/8 for the load sample causes the red blood cells to stream. For the non-bound solution sample, a dilution of greater than 1/3 gives streaming of the blood cells. It can be concluded from this assay that the A-antigen adsorbent (Scaffold Example 1) gives a 1/8 to 1/3 clearance of isoagglutinin from the feed.

### EXAMPLE 4

### Removal of B-isoagglutinin from human IVIG intermediate product feedstock using a product of this invention

### 4.1 Column chromatography purification of IVIG feedstock

A column containing the antigen B ligand (Scaffold Example 2) is packed followed by the application of an IVIG solution containing isoagglutinins at a flow rate of 1 mL/min. The flow-through from the loading process is collected. The load and flow-through samples are analysed for the content of B-isoagglutinins using a standard agglutination assay.

### 4.2 Agglutination assay on flow through from column chromatography purification of IVIG feedstock with B-antigen beaded agarose adsorbent

### 4.2.1 Preparation of red blood cells

To make the type-B red blood cells up in PBS buffer, the cells are washed with PBS buffer by adding 2mL red blood cells into a 5 mL centrifuge tube and centrifuging at 1400 rpm for 3 minutes. The supernatant from the centrifuged cells is removed before adding PBS buffer to the cell pellet to make the suspension up to a 2 mL volume, and the cells are mixed by inverting the tube. This PBS wash is repeated three times.

Freeze dried papain was reconstituted with PBS buffer (2 mL) before adding 200 µL to the cells after centrifugation and removal of the supernatant. These suspensions are then made up to 2 mL volume with PBS buffer before incubation with mixing for 10 minutes at 37 °C.

At the end of this incubation period, the suspension is centrifuged for 3 minutes at 1400 rpm. The supernatant is removed from the centrifuged cells, then PBS buffer added to make the suspension up to a 2 mL volume. The cells are mixed by inverting the tube. This PBS wash is repeated three times.

The spun cells are mixed with inversion made up to 2 mL volume with 2 mg/mL BSA solution.

### 4.2.2 Performing the agglutination test

A sample of the feedstock from step 4.1 is diluted 1/2, 1/4, 1/6, 1/8, 1/10, 1/12, 1/14 and 1/16 with a solution of 2 mg/mL BSA solution.

A sample of the non-bound from step 4.1 is diluted 1/2, 1/3, 1/4, 1/5, 1/6, 1/7, 1/8 and 1/9 with a solution of 2 mg/mL BSA solution.

The feedstock and non-bound dilutions described above (20 µL) are transferred to a 96 well v-bottomed plate along with the blank 2mg/mL BSA solution.

The red blood cells prepared in stage 4.2.1 are mixed by inversion before transferring to a pipette reservoir. The blood cell suspension (20 µL) is transferred to the plate using a multichannel pipette. The plate is agitated to mix the solutions for 30 seconds before centrifuging the plate at 1400 rpm for 3 minutes.

The plate is placed onto a stand at a 70° angle for 15 minutes. Each well of the plate is studied to determine the level of clearance of isoagglutinin from the red blood cells. This is achieved by assessing the sample dilution required to prevent agglutination, indicated by streaming of the red blood cells in the plate wells (Figure 3).

As shown in Figure 3, a dilution of greater than 1/4 for the load sample causes the red blood cells to stream. For the non-bound solution sample, any dilution gives streaming of the blood cells. It can be concluded from this assay that the B-antigen adsorbent (Scaffold Example 2) gives a 1/4 to neat clearance of isoagglutinin from the feed.

### EXAMPLE 5

### Caustic stability of products of the invention

### 5.1 Stability of an A-Antigen adsorbent

Two samples of the A-Antigen adsorbent were incubated in 0.5 M NaOH at 40 °C for 1 week, and subsequently tested for performance using a standard agglutination assay. The samples both showed reduction in agglutinin titres of 1/10 to 1/4 before and after incubation under caustic conditions, indicating the stability of the adsorbent under those conditions.

In another experiment, a batch of the A-Antigen adsorbent was subjected to a cycling study, where a packed column of the adsorbent went through 51 cycles of a procedure containing a caustic-based cleaning-in-place (CIP) step using 0.5 M NaOH. The isoagglutinin titre of samples taken before and after the column run both showed a reduction of agglutinin titre from 1/8 to 1/2 after the first and 51^{st} runs, demonstrating the stability of the material to caustic conditions.

### 5.2 Stability of a B-Antigen adsorbent

A batch of the B-Antigen adsorbent was subjected to a cycling study, where a packed column of the adsorbent went through 51 cycles of a procedure containing a caustic-based cleaning-in-place (CIP) step using 0.5 M NaOH. The isoagglutinin titre of samples taken before and after the column run both showed a reduction of agglutinin titre from 1/4 to 1/1 (neat) after the first and 51^{st} runs, demonstrating the stability of the material to caustic conditions.

### EXAMPLE 6

### Functionalisation of an activated base matrix

### 6.1 Coupling an antigen-A trisaccharide ligand to DCT-activated base matrix at varying activation densities

A range of DCT activated substrates with activation densities ranging from 30 µmol/g to 140 µmol/g were generated using the method described in Example 1.

A solution of A-antigen trisaccharide ligand comprising a flexible linker in water was added to the DCT gel slurries maintained under high pH at room temperature for 24 hours. The A-antigen trisaccharide ligand comprising the flexible linker is a compound of formula (IVa), as described herein. After 0, 0.5, 1, 2, 3, 5 and 24 hours, samples of the reaction supernatants were collected for quantitation of unreacted ligand.

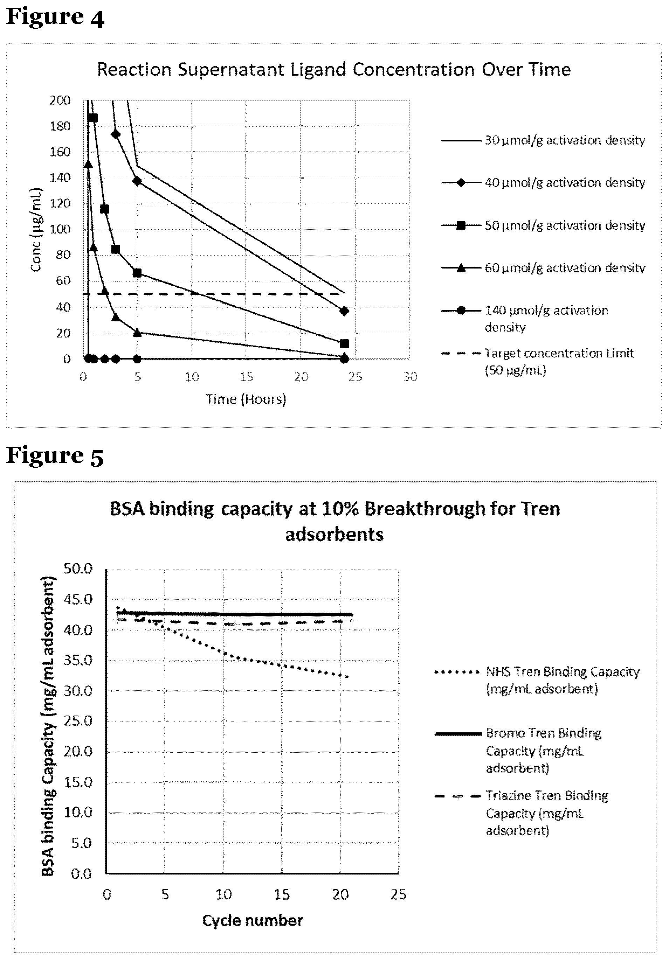

As shown in Figure 4, higher activation densities afforded by the invention allow coupling of the ligand to the target concentration within 5-hours reaction time. It can be concluded that activation densities equal to or lower than 40 µmol/g would fail to meet the target immobilisation level within 24 hours. Other activation methods such as epichlorohydrin activation (Example 12) are not capable of achieving activation densities higher than 30 µmol/g settled on a beaded agarose support.

### EXAMPLE 7 (Comparative)

A synthetic route for producing a n-hydroxy succinimide scaffold is shown in Scheme 1. The steps are described in more detail below.

### Solid phase activation of a solid support

### 7.1 Carboxy activation

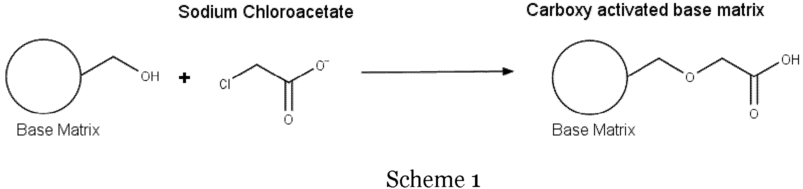

Approximately 1 kg of a solid support, such as beaded agarose, is suspended in water before heating to 40 to 50 °C. Sodium chloroacetate (approximately 235 g) is added to the reaction slurry and left to react for no more than 18 hours, after which the drained gel is washed with water. The resulting material is an activated matrix that contains 20 to 30 µmol carboxy groups per g adsorbent.

### 7.2 N-hydroxy succinimide esterification

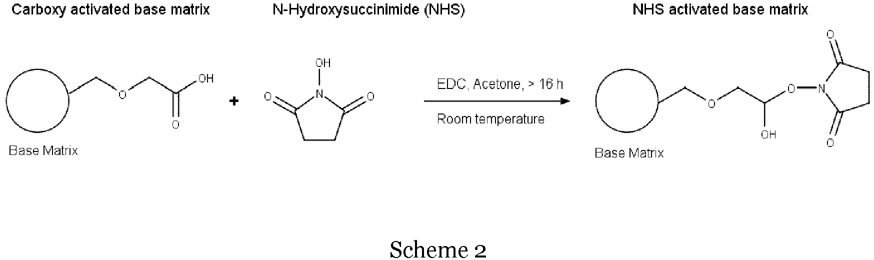

The carboxylated support is acidified by washing with 0.1 M HCl and then washed free of water with acetone. The gel is then suspended in acetone before reacting with N-hydroxysuccinimide (NHS) (16 g per kg of support) and *N*-ethyl-*N*'-(3-(dimethylamino)propyl)carbodiimide (EDC) (10 g per kg of support) at ambient temperature for at least 16 hours. After the reaction, the gel is drained and washed with *N,N*-dimethylformamide. The resulting material is an activated matrix that contains 20 to 30 µmol NHS groups per g adsorbent.

### EXAMPLE 8

### Functionalisation of a base matrix with tris 2-aminoethyl amine ligand

### 8.1 Coupling tris 2-aminoethyl amine ligand to brominated base matrix (Comparative Scaffold 1)

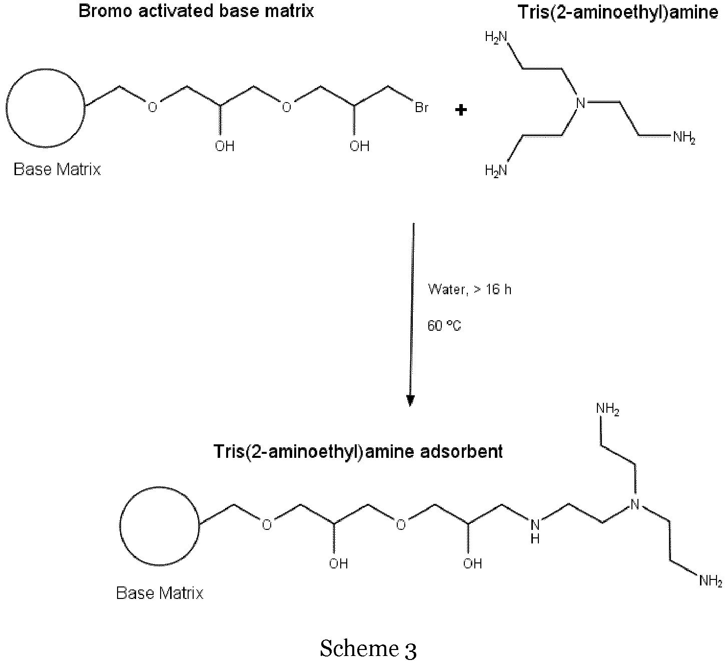

The bromo activated base matrix produced as described in Example 1 is suspended in water before adding tris 2-aminoethyl amine ligand. The charge of tris 2-aminoethyl ligand is calculated with respect to the precursor allyl activation density to give an excess of 30 molar equivalents.

After addition of the amine, the reaction is left to react at 60 °C for at least 16 hours. At the end of the reaction period, the gel is drained and washed with water, 0.1 M HCl and 0.1 M NaCl before settling under gravity.

This reaction results in a chromatographic material containing an affinity ligand which binds bovine serum albumin.

### 8.2 Coupling tris 2-aminoethyl) amine ligand to NHS activated base matrix

### (Comparative Scaffold 2)

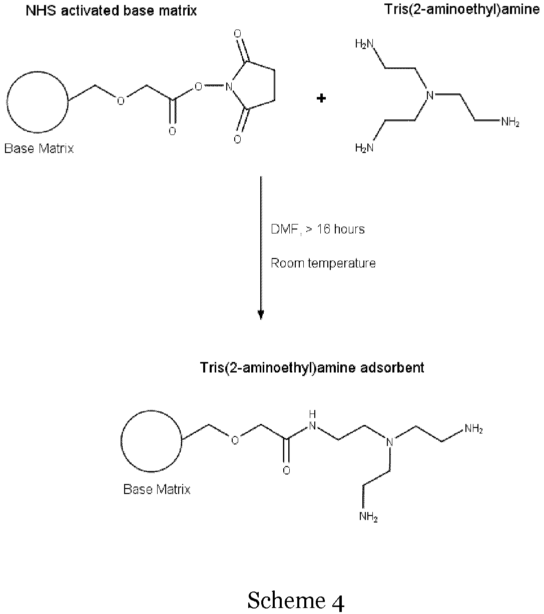

The NHS activated base matrix produced as described in Example 7 is suspended in N,N-dimethylformamide before adding tris 2-aminoethyl ligand . The charge of tris 2-aminoethyl ligand is calculated with respect to the precursor NHS activation density to give an excess of 30 molar equivalents.

After addition of the amine, the reaction is left to react at ambient temperature for at least 16 hours. At the end of the reaction period, the gel is drained and washed with water, 0.1 M HCl and 0.1 M NaCl before settling under gravity.

This reaction results in a chromatographic material containing an affinity ligand which binds bovine serum albumin.

### 8.3 Coupling tris 2-aminoethyl) amine ligand to DCT activated base matrix (Scaffold

### Example 3)

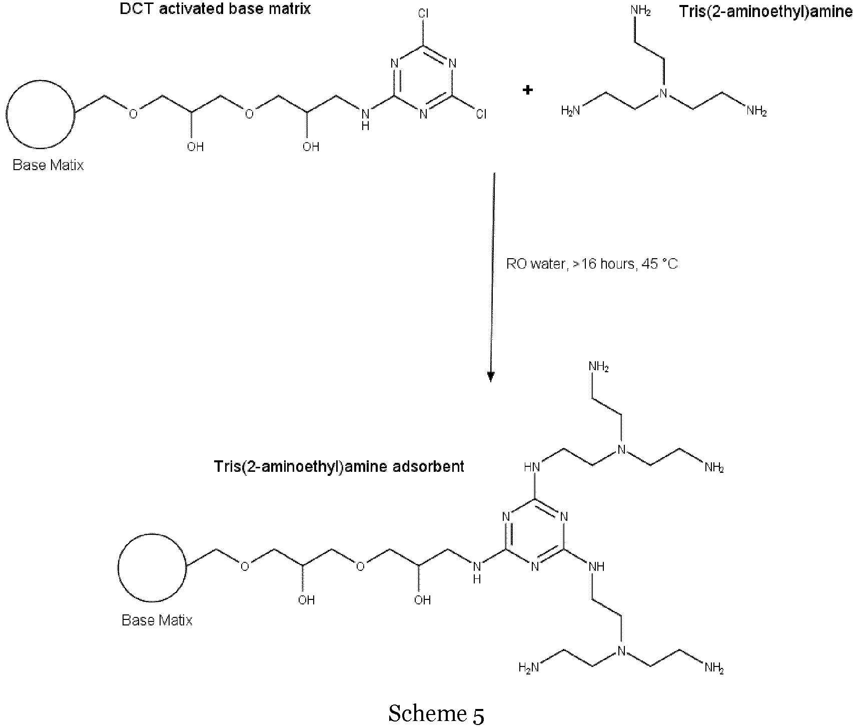

The DCT activated base matrix produced as described in Example 1 is suspended in water before adding tris 2-aminoethyl ligand. The amount of tris 2-aminoethyl ligand is calculated with respect to the precursor activation density to give an excess of 10 molar equivalents.

After addition of the amine, the reaction is left to react at 45 °C temperature for at least 16 hours. At the end of the reaction period, the gel is drained and washed with water, 0.1 M HCl and 0.1 M NaCl before settling under gravity.

This reaction results in a chromatographic material containing an affinity ligand which binds bovine serum albumin.

### EXAMPLE 9 Caustic stability of tris 2-aminoethyl ligand adsorbent

### 9.1 Stability of tris 2-aminoethyl amine (tren) adsorbents

A sample of the tren adsorbent produced as described in Example 8.1 (bromo attachment, Comparative Scaffold 1), 8.2 (NHS attachment, Comparative Scaffold 2) and 8.3 (DCT attached product of the invention, Scaffold Example 3) were subjected to a cycling study, where packed columns of the adsorbents went through 21 cycles of a procedure containing a caustic-based cleaning-in-place (CIP) step using 0.5 M NaOH. On the first, 11^{th} and 21^{st} cycle, the column was loaded to 10% breakthrough with bovine serum albumin (BSA) and the binding capacity was calculated.

As shown in Figure 5, the BSA binding capacity of the Scaffold Example 3 adsorbent at 10% break through (approximately 41 mg/mL) did not change significantly from cycle 1 to 21, demonstrating stability of the Scaffold Example 3 to caustic conditions. In contrast, binding capacity of Scaffold Example 2 fell significantly under the same conditions.

The bromo attached Tren adsorbent of Comparative Scaffold 1 also showed good caustic stability however, as described in Example 8.1 this required much stronger reaction conditions compared to the triazine attachment process for Scaffold Example 3, including higher temperature and a three-fold excess of amine (Tren ligand).

### EXAMPLE 10

### Functionalisation of activated scaffolds with Blue chromophore ligand 10.1 Coupling Blue chromophore ligand to brominated base matrix (Comparative Scaffold 3)

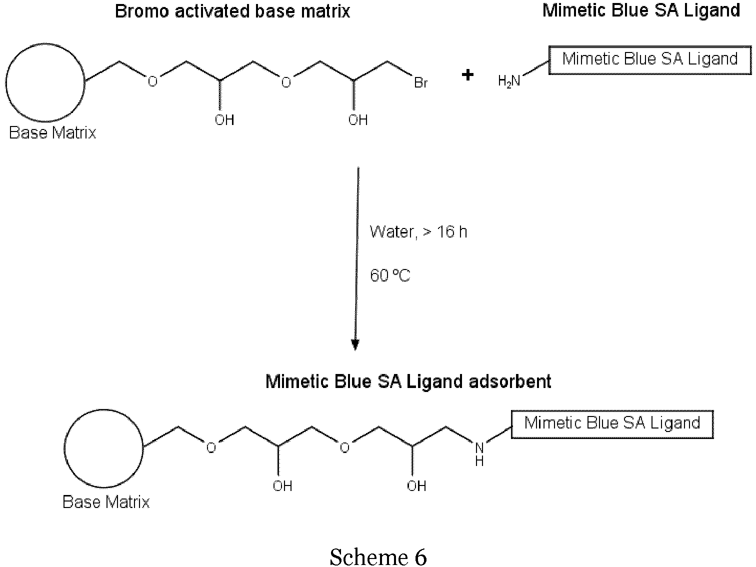

A solution of 4.5 g of Mimetic Blue SA ligand available from Astrea Bioseparations (referred to herein as "Blue ligand") per kg of adsorbent is prepared in water with adjustment to pH 12 with NaOH. The bromo activated base matrix produced as described in Example 1 is suspended in the Blue ligand solution and the reaction is left to react at 60 °C for at least 16 hours. At the end of the reaction period, any residual reactive sites on the gel are blocked by addition of ethanolamine followed by reaction at 60 °C for at least 16 hours. After the blocking reaction, the gel is drained and washed with water. This reaction results in a chromatographic material containing an affinity ligand at approximately 3.0 µmol ligand per g of adsorbent which binds human serum albumin.

### 10.2 Coupling Blue chromophore ligand to NHS activated base matrix (Comparative Scaffold 4)

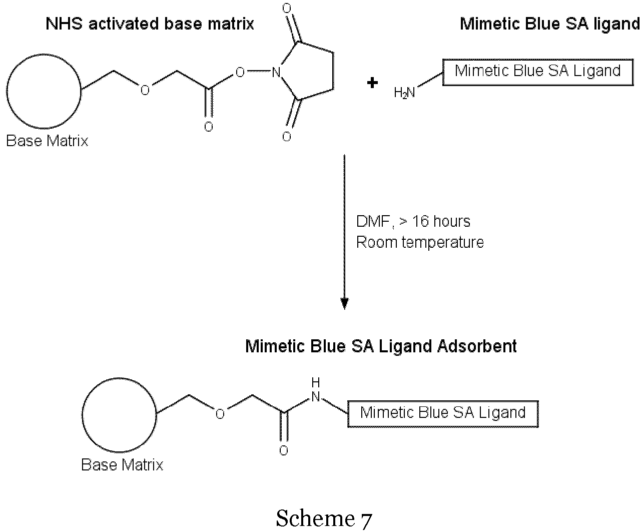

A solution of 4.5 g of Blue ligand per kg of adsorbent is prepared in water with adjustment to pH 12 with NaOH. The NHS activated base matrix produced as described in Example 7 is suspended in the Blue ligand solution and the reaction is left to react at ambient temperature for at least 16 hours. At the end of the reaction period, any residual reactive sites on the gel are blocked by addition of ethanolamine followed by reaction at room temperature for at least 16 hours. After the blocking reaction, the gel is drained and washed with water. This reaction results in a chromatographic material containing an affinity ligand at approximately 3.4 µmol ligand per g of adsorbent which binds human serum albumin.

### 10.3 Coupling Blue chromophore ligand to DCT activated base matrix (Scaffold

### Example 4)

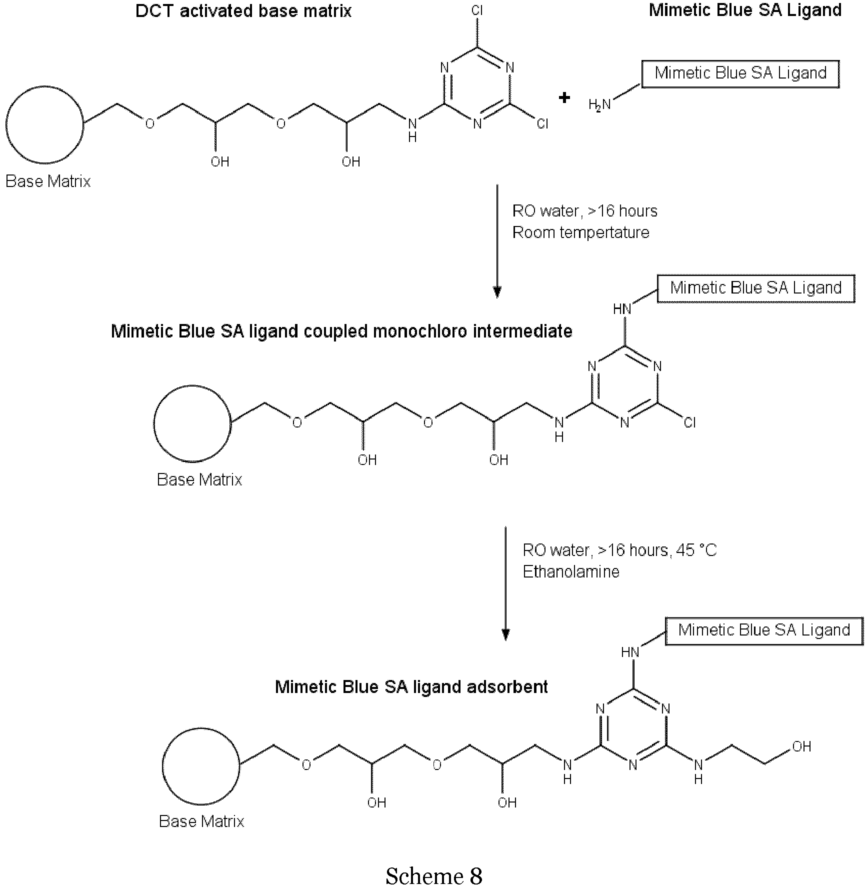

A solution of 4.5 g of Blue ligand per kg of adsorbent is prepared in water with adjustment to pH 12 with NaOH. The DCT activated base matrix produced as described in Example 1 is suspended in the Blue ligand solution and the reaction is left to react at ambient temperature for at least 16 hours. At the end of the reaction period, any residual reactive sites on the gel are blocked by addition of ethanolamine followed by reaction at 45 °C for at least 16 hours. After the blocking reaction, the gel is drained and washed with water. This reaction results in a chromatographic material containing an affinity ligand at approximately 6 µmol ligand per g of adsorbent which binds human serum albumin.

### EXAMPLE 11

### Caustic stability of Blue ligand scaffolds

A sample of the Blue chromophore ligand scaffolds produced as described in Example 10.1 (bromo attachment, Comparative Scaffold 3), 10.2 (NHS attachment, Comparative Scaffold 4) and 10.3 (DCT attached product of the invention, Scaffold Example 4) were subjected to a cycling study, where a packed column of the adsorbents went through 21 cycles of a procedure containing a caustic-based cleaning-in-place (CIP) step using 0.5 M NaOH. On the first, 11^{th} and 21^{st} cycle, the columns were loaded to 10% breakthrough with human serum albumin (HSA) and the binding capacity was calculated.

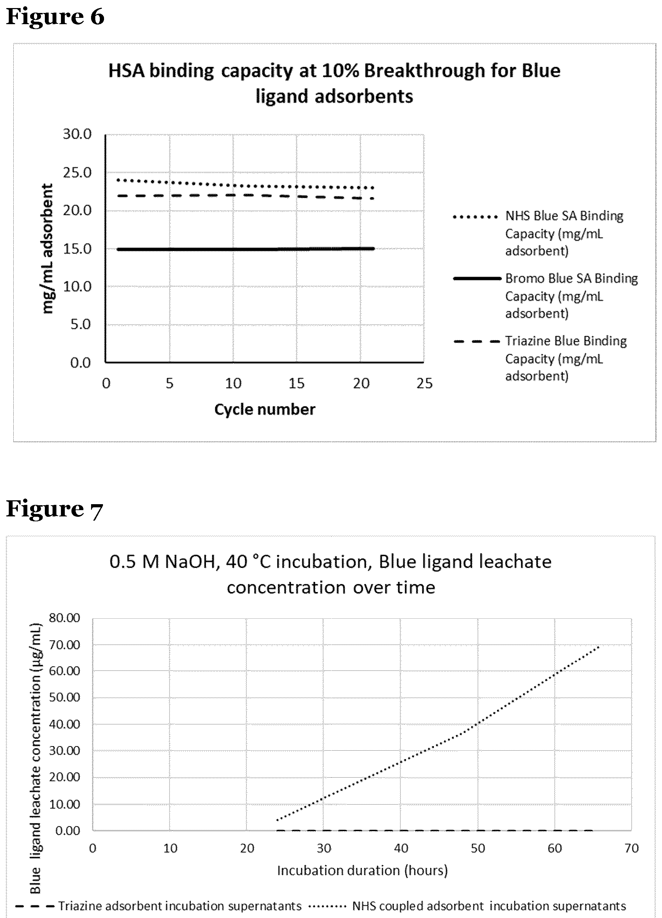

As shown in Figure 6, the HSA binding capacity of the DCT coupled adsorbent (Scaffold Example 4) and bromo coupled adsorbent (Comparative Scaffold 3) at 10% break through did not change significantly from cycle 1 to 21, demonstrating stability of the adsorbents to caustic conditions. The NHS coupled comparative example (Comparative Scaffold 4) showed slight decrease in binding capacity by the 21^{st} cycle.

Despite being activated to the same density and having the same excess of ligand in the immobilisation reaction, the triazine adsorbent showed higher binding capacity than the bromo attached adsorbent. Without wishing to be bound by any theory, this is afforded by the higher reactivity of the chlorotriazine reactive group leading to increased ligand density of the adsorbent and demonstrating an advantage in efficiency of the synthesis process.

In another experiment, a sample of the Blue chromophore ligand adsorbents produced as described in Example 10.2 (NHS coupled Comparative Scaffold 4) and 10.3 (DCT attached product of the invention Scaffold Example 4), were incubated in 0.5 M NaOH at 40 °C for three days. Approximately every 24 hours, a supernatant sample was collected for quantitation of ligand leachate concentration by HPLC.

As shown in Figure 7, this analysis showed negligible levels of ligand leachate in the incubation supernatant of the triazine coupled Scaffold Example 4 demonstrating the stability of the product to caustic conditions. Conversely, the NHS coupled Comparative Scaffold 4 showed significant levels of Blue ligand leachate increasing over the course of the incubation, indicating a lack of caustic stability.

The two adsorbents were tested for HSA binding capacity at 10% breakthrough before the stability study and after incubation in 0.5 M NaOH at 40 °C for 66 hours.

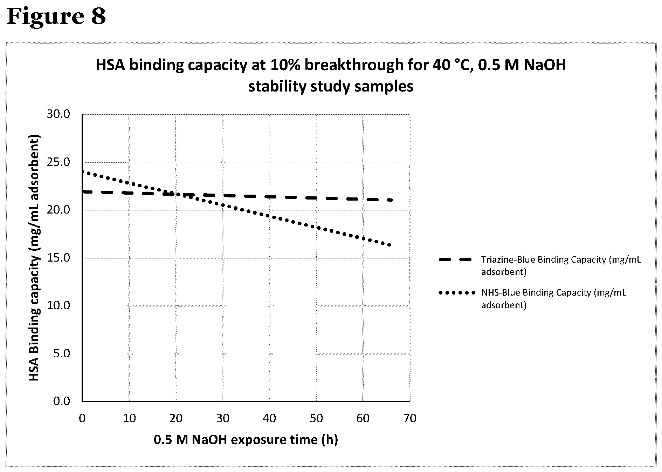

As shown in Figure 8, the triazine coupled Scaffold Example 4 showed negligible change in binding capacity after exposure to 0.5 M NaOH at 40 °C for 66 hours. This demonstrates the caustic stability of the product. For the NHS coupled Comparative Scaffold 4, binding capacity significantly dropped over the 66-hour incubation period demonstrating the poor caustic stability of this attachment method.

### EXAMPLE 12 (Comparative)

### 12.1 Activation of beaded agarose with epichlorohydrin

Approximately 1 kg of a beaded agarose solid support, is suspended in 0.9 L of water before addition of 166 mL of 10 M NaOH. Epichlorohydrin is added to the reaction

slurry at 250 mL per kg of base matrix. The reaction is left to stir at 16 °C for at least 16 hours before adding an additional 125 mL portion of epichlorohydrin and 84 mL of 10 M NaOH. The reaction mixture is left to react for 3 hours before draining and washing with water. The resulting material is an activated matrix that contains up to 30 µmol epoxide groups per g adsorbent, demonstrating the limited activation density of epichlorohydrin activation method on beaded agarose support.

### Summary

The inventors have shown that their functionalised solid support can be used to selectively isolate isoagglutinin. However, it will be appreciated that the scaffold could be functionalised with alternative ligands to enable the isolation of other biomolecules.

The inventors have also shown that the functionalised solid support is compatible with cleaning and sanitisation using sodium hydroxide, thereby preventing contamination thereof. The functionalised solid support is stable under these conditions, allowing repeated use thereof. This will significantly reduce the cost of isolating biomolecules, and in turn any products manufactured therewith.

In addition, the inventors have shown that their activated solid support can be functionalised under mild reaction conditions (low temperature, low molar excess of amine and short reaction times) by virtue of the high activation densities achievable by this invention and the high reactivity of the dichlorotriazine attachment chemistry. It will also be appreciated by those skilled in the art that this high potential activation density enables higher ligand densities which in turn can provide increased binding capacity of target biomolecules.

The invention may be as defined by the following clauses:
1. A method of producing an activated substrate, the method comprising:
   (a) modifying a substrate, wherein the substrate comprises a base matrix, to form a base matrix comprising a leaving group;
   (b) contacting the base matrix formed in step (a) with an aminating agent, to thereby provide an aminated base matrix; and
   (c) contacting the aminated base matrix formed in step (b) with a heteroaromatic compound, wherein the heteroaromatic compound is a 5 to 12 membered heteroaromatic ring substituted with at least two halogens and optionally one or more further substituents, to produce an activated substrate.
2. The method of clause 1, wherein the substrate comprises a nucleophilic moiety, which is preferably a hydroxyl moiety.
3. The method of clause 1 or clause 2, wherein step (a) of the method comprises:
   ai) contacting the substrate with an electrophile, wherein the electrophile comprises an unsaturated hydrocarbon chain, to thereby form a base matrix comprising an unsaturated hydrocarbon chain; and
   aii) contacting the base matrix formed in step (ai) with a halogenating agent, to thereby provide a halogenated base matrix.
4. The method of clause 3, wherein the electrophile is a compound of formula (I):

   R¹-L¹-X¹-L²-R² (I)

   , wherein R¹ is an optionally substituted 3 to 6 membered heterocyclic ring or a leaving group;
   R² is an optionally substituted C₂-C₁₂ alkenyl or an optionally substituted C₂-C₁₂ alkynyl;
   L¹ and L² are each independently absent, an optionally substituted C₁₋₁₂ alkylene, an optionally substituted C₂₋₁₂ alkenylene or an optionally substituted C₂₋₁₂ alkynylene,
   where the backbone of the alkylene, alkenylene or alkynlene is optionally interrupted by one or more heteroatoms;
   X¹ is NR³, O or S; and
   R³ is H, an optionally substituted C₁₋C₆ alkyl, an optionally substituted C₂-C₆ alkenyl or an optionally substituted C₂-C₆ alkynyl.
5. The method of clause 3 or clause 4, wherein the halogenating agent is N-bromosuccinimide, N-chlorosuccinimide, bromine or chlorine.
6. The method of any preceding clause, wherein the aminating agent is NH₂R¹⁶ or 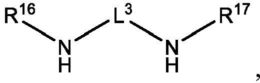 wherein R¹⁶ and R¹⁷ are independently H, an optionally substituted C₁₋₂₄ alkyl, an optionally substituted C₂₋₂₄ alkenyl or an optionally substituted C₂₋₂₄ alkynyl and L³ is an optionally substituted C₁₋₁₂ alkylene, an optionally substituted C₂₋₁₂ alkenylene or an optionally substituted C₂₋₁₂ alkynylene, where the backbone of the alkylene, alkenylene or alkynlene is optionally interrupted by one or more heteroatoms.
7. The method of any preceding clause, wherein the heteroaromatic compound is a 5 or 6 membered heteroaromatic ring which is substituted with at least two halogens, and optionally one or more further substituents independently selected from the group consisting of OH, SH, COOH, NH₂, optionally substituted C₁-C₂₄ alkyl, optionally substituted C₂-C₂₄ alkenyl and optionally substituted C₂-C₂₄ alkynyl.
8. The method of clause 7, wherein the heteroaromatic compound is dichlorotriazine or cyanuric chloride.
9. The activated substrate obtained or obtainable by the method of any one of clauses 1 to 8.
10. An activated substrate of formula (II): 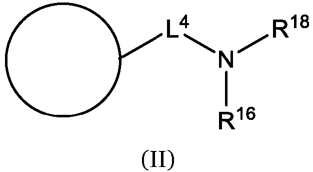
   , wherein L⁴ is an optionally substituted C₁₋₂₄ alkylene, an optionally substituted C₂₋₂₄ alkenylene or an optionally substituted C₂₋₂₄ alkynylene, where the backbone of the alkylene, alkenylene or alkynlene is optionally interrupted by one or more heteroatoms; R¹⁶ is H, an optionally substituted C₁₋₂₄ alkyl, an optionally substituted C₂₋₂₄ alkenyl or an optionally substituted C₂₋₂₄ alkynyl; and
   R¹⁸ is a 5 to 12 membered heteroaryl group substituted with at least one halogen, and optionally substituted with one or more further substituents.
11. The activated substrate of clause 10, wherein R¹⁸ is 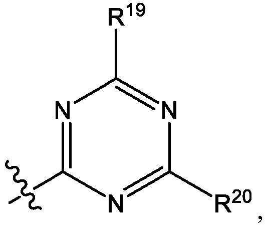 wherein R¹⁹ is a halogen and R²⁰ is a halogen, H, OH, SH, COOH, NH₂, optionally substituted C₁-C₂₄ alkyl, optionally substituted C₂-C₂₄ alkenyl or optionally substituted C₂-C₂₄ alkynyl.
12. The activated substrate of clause 10 or 11 wherein L⁴ is substituted by at least two hydroxyl groups.
13. The activated substrate of any one of clauses 10-12 wherein L⁴ is a C₁₋₂₄ alkylene, an optionally substituted C₂₋₂₄ alkenylene or an optionally substituted C₂₋₂₄ alkynylene interrupted by at least two O atoms.
14. The activated substrate of any one of clauses 10-13, wherein the compound of formula (I) is a compound of formula (IIb): 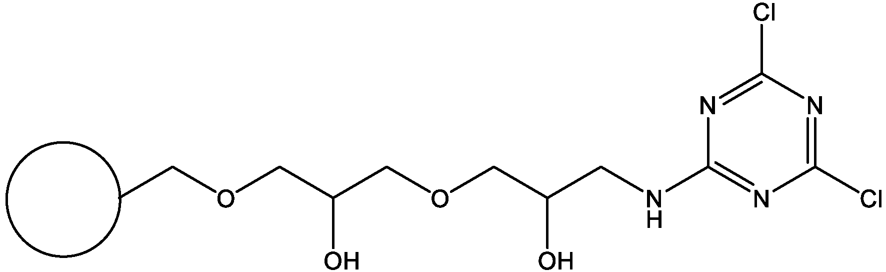 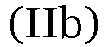
15. A method of producing a scaffold for isolation of a biomolecule, the method comprising:
   - conducting the method of any one of clauses 1 to 8 to produce an activated substrate, or providing the activated substrate of any one of clauses 9 to 14; and
   - contacting the activated substrate with a molecule comprising a ligand specific for a biomolecule to provide a scaffold for isolation of the biomolecule.
16. The method of clause 15, wherein the biomolecule is selected from the group consisting of an amino acid, an aptamer, a peptide, an affimer, a protein, a glycoprotein, a lipopolysaccharide, an antibody or a fragment thereof, a nucleic acid, an organic polymer, a virus, a bacterium, a cell, and a cell-related structure.
17. The method according to clause 15 or clause 16, wherein the molecule comprising the ligand is a compound of formula (IV):

   X₄-L₅-X₅ (IV)

   , wherein X⁴ is NH₂, SH or OH;
   L⁵ is absent or is an optionally substituted C₁₋₃₀ alkylene, an optionally substituted C₂₋₃₀ alkenylene or an optionally substituted C₂₋₃₀ alkynylene, where the backbone of the alkylene, alkenylene or alkynlene is optionally interrupted by one or more heteroatoms; and
   X⁵ is a ligand specific for a biomolecule.
19. The method according to clause 17 wherein X⁵ comprises an NH₂ group, a boronate group or a naphthol group.
19. The method of any one of clause 15 to 17, wherein the ligand comprises an optionally derivatized saccharide molecule, an optionally derivatized amino acid, an optionally derivatized peptide, an optionally derivatized affimer or an optionally derivatized protein, and preferably comprises an optionally derivatized saccharide molecule.
20. The method of clause 19, wherein the compound comprising the ligand is a compound of Formula (IVa) or (IVb): 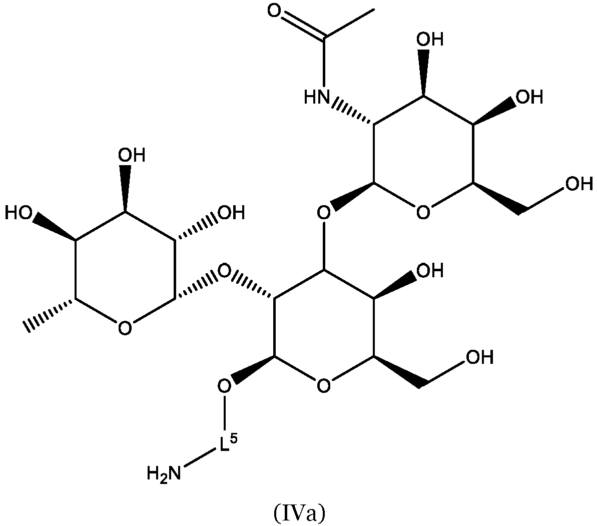 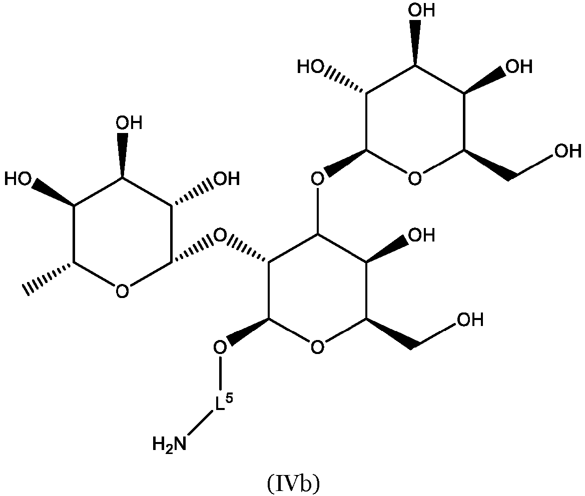
21. The method of any one of clauses 15 to 20, wherein the method subsequently comprises contacting the scaffold with an alcohol, a hydroxide, ammonia, an amine or a thiol.
22. A scaffold for isolation of a biomolecule obtained or obtainable by the method of any one of clauses 15 to 21.
23. A scaffold for isolation of a biomolecule of formula (III): 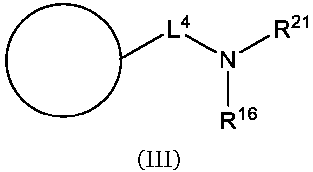
   , wherein L⁴ and R¹⁶ are as defined in any one of clauses 10-14; and
   R²¹ is a 5 to 12 membered heteroaryl group substituted with at least one group comprising a ligand specific for a biomolecule, and optionally substituted with one or more further substituents.
24. The scaffold of clause 23, wherein R²¹ is 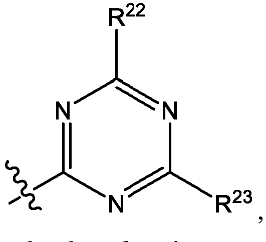 wherein R²² is a group comprising a ligand specific for a biomolecule and R²³ is a group comprising a ligand specific for a biomolecule, a halogen, H, OR²⁴, COOR₂₄, NR₂₄R₂₅, SR₂₄, optionally substituted C₁-C₂₄ alkyl, optionally substituted C₂-C₂₄ alkenyl or optionally substituted C₂-C₂₄ alkynyl, wherein R²⁴ and R²⁵ are independently H optionally substituted C₁-C₂₄ alkyl, optionally substituted C₂-C₂₄ alkenyl or optionally substituted C₂-C₂₄ alkynyl.
25. The scaffold according to clause 23 or 24 wherein a concentration of the ligand of the scaffold is at least 40 µmol/g.
26. The scaffold of any one of clauses 23-25, wherein the or each group comprising the ligand specific for the biomolecule is 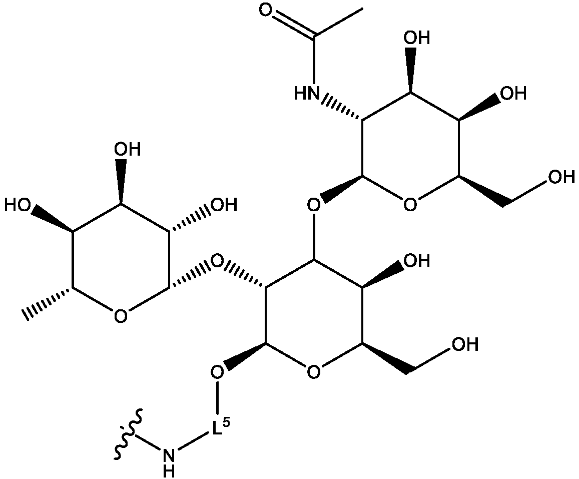 or 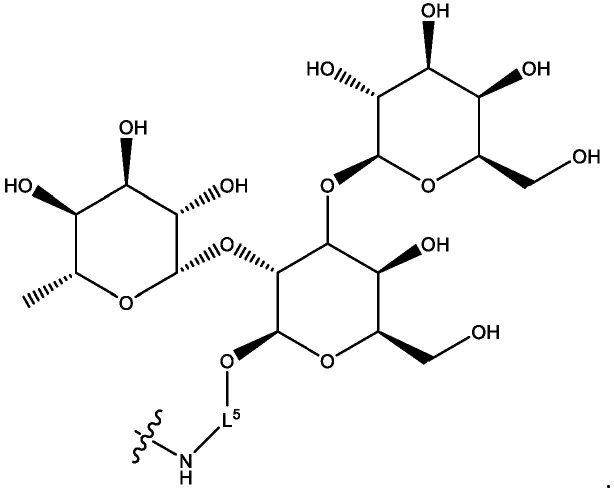
27. Use of the scaffold of any one of clauses 22 to 26 isolate a biomolecule.
28. A method of isolating a biomolecule on a scaffold, the method comprising contacting a scaffold with a biomolecule, wherein the scaffold is as defined in any one of clauses 22 to 26.
29. The method according to clause 28 wherein the biomolecule is selected from isoagglutinins, lipopolysaccharides, albumins, glycosylated proteins and insulin.
30. A method of cleaning a scaffold, the method comprising contacting a scaffold with a caustic substance, wherein the scaffold is as defined in any one of clauses to 26.

## Claims

1. A method of producing an activated substrate, the method comprising:
(a) modifying a substrate, wherein the substrate comprises a base matrix, to form a base matrix comprising a leaving group, wherein modifying the substrate comprises:
(ai) contacting the substrate with an electrophile, wherein the electrophile comprises an unsaturated hydrocarbon chain, to thereby form a base matrix comprising an unsaturated hydrocarbon chain; and
(aii) contacting the base matrix formed in step (ai) with a halogenating agent, to thereby provide a halogenated base matrix;
(b) contacting the base matrix formed in step (a) with an aminating agent, to thereby provide an aminated base matrix; and
(c) contacting the aminated base matrix formed in step (b) with a heteroaromatic compound, wherein the heteroaromatic compound is a 5 to 12 membered heteroaromatic ring substituted with at least two halogens and optionally one or more further substituents, to produce an activated substrate.

2. The method of claim 1, wherein the substrate comprises a nucleophilic moiety, which is preferably a hydroxyl moiety.

3. The method of claim 1 or claim 2, wherein the electrophile is a compound of formula (I):
R₁-L¹-X¹-L²-R² (I)
, wherein R¹ is an optionally substituted 3 to 6 membered heterocyclic ring or a leaving group;
R² is an optionally substituted C₂-C₁₂ alkenyl or an optionally substituted C₂-C₁₂ alkynyl;
L¹ and L² are each independently absent, an optionally substituted C₁₋₁₂ alkylene, an optionally substituted C₂₋₁₂ alkenylene or an optionally substituted C₂₋₁₂ alkynylene, where the backbone of the alkylene, alkenylene or alkynlene is optionally interrupted by one or more heteroatoms;
X¹ is NR³, O or S; and
R³ is H, an optionally substituted C₁₋C₆ alkyl, an optionally substituted C₂₋C₆ alkenyl or an optionally substituted C₂-C₆ alkynyl.

4. The method of any preceding claim, wherein the halogenating agent is N-bromosuccinimide, N-chlorosuccinimide, bromine or chlorine.

5. The method of any preceding claim, wherein the aminating agent is NH₂R¹⁶ or 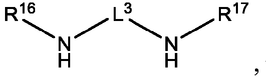 wherein R¹⁶ and R¹⁷ are independently H, an optionally substituted C₁₋₂₄ alkyl, an optionally substituted C₂₋₂₄ alkenyl or an optionally substituted C₂₋₂₄ alkynyl and L³ is an optionally substituted C₁₋₁₂ alkylene, an optionally substituted C₂₋₁₂ alkenylene or an optionally substituted C₂₋₁₂ alkynylene, where the backbone of the alkylene, alkenylene or alkynlene is optionally interrupted by one or more heteroatoms.

6. The method of any preceding claim, wherein the heteroaromatic compound is a 5 or 6 membered heteroaromatic ring which is substituted with at least two halogens, and optionally one or more further substituents independently selected from the group consisting of OH, SH, COOH, NH₂, optionally substituted C₁-C₂₄ alkyl, optionally substituted C₂-C₂₄ alkenyl and optionally substituted C₂-C₂₄ alkynyl.

7. The method of claim 6, wherein the heteroaromatic compound is dichloro-triazine or cyanuric chloride.

8. A method of producing a scaffold for isolation of a biomolecule, the method comprising:
- conducting the method of any one of claims 1 to 7 to produce an activated substrate; and
- contacting the activated substrate with a molecule comprising a ligand specific for a biomolecule to provide a scaffold for isolation of the biomolecule.

9. The method of claim 8, wherein the biomolecule is selected from the group consisting of an amino acid, an aptamer, a peptide, an affimer, a protein, a glycoprotein, a lipopolysaccharide, an antibody or a fragment thereof, a nucleic acid, an organic polymer, a virus, a bacterium, a cell, and a cell-related structure.

10. The method of claim 9, wherein the ligand specific for a biomolecule is a compound of formula (IVc):
H₂N-L⁵-N(Ak-NH₂)₂ (IVc)
wherein L⁵ is absent or is an optionally substituted C₁₋₃₀ alkylene, an optionally substituted C₂₋₃₀ alkenylene or an optionally substituted C₂₋₃₀ alkynylene where the backbone of the alkylene, alkenylene or alkynlene is optionally interrupted by one or more heteroatoms, or an optionally substituted C₆₋₂₀ arylene; and
Ak in each occurrence is a C₁₋₁₂ alkylene where, in the case of a C₂₋₁₂ alkylene, the backbone of the alkylene is optionally interrupted by one or more heteroatoms, e.g one or more O atoms.

11. A scaffold for isolation of a biomolecule of formula (III): 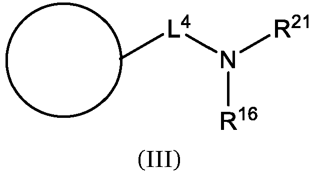
, wherein L⁴ is a C₁₋₂₄ alkylene, a C₂₋₂₄ alkenylene or a C₂₋₂₄ alkynylene, where the backbone of the alkylene, alkenylene or alkynylene is optionally interrupted by one or more heteroatoms and L⁴ is substituted with at least two hydroxyl groups;
R¹⁶ is H, an optionally substituted C₁₋₂₄ alkyl, an optionally substituted C₂₋₂₄ alkenyl or an optionally substituted C₂₋₂₄ alkynyl; and
R²¹ is a 5 to 12 membered heteroaryl group substituted with at least one group comprising a ligand specific for a biomolecule, and optionally substituted with one or more further substituents, wherein the ligand specific for the biomolecule is as defined in claim 10.

12. The scaffold according to claim 12, wherein the scaffold has the formula:
